# NEW EUROPEAN PATENT SPECIFICATION

(11) **EP 1 097 196 B2**
(45) Date of publication and mention of the opposition decision: **20.04.2011**
(45) Mention of the grant of the patent: 19.09.2007
(21) Application number: 99931029.5
(22) Date of filing: 09.07.1999
(51) Int. Cl.: C12N 9/34

(54) **GLUCOAMYLASE VARIANTS**
VARIANTEN DER GLUKOAMYLASE
VARIANTS DE GLUCOAMYLASE

(30) Priority: 15.07.1998 WO PCT/DK98/00937; 17.12.1998 WO PCT/DK98/01667
(43) Date of publication of application: 09.05.2001
(60) Divisional application: 07116129.3
(73) Proprietor: Novozymes A/S, 2880 Bagsvaerd (DK)
(72) Inventor: NIELSEN, Bjarne, Ronfeldt, DK-2830 Virum (DK); SVENDSEN, Allan, DK-3460 Birkerod (DK); PEDERSEN, Henrik, DK-5210 Odense SV (DK); VIND, Jesper, DK-2800 Lyngby (DK); HENDRIKSEN, Hanne, Vang, DK-2840 Holte (DK); FRANDSEN, Torben, Peter, DK-1826 Frederiksberg C (DK)
(86) International application number: PCT/DK1999/000392
(87) International publication number: WO 2000/004136

(56) References cited:
- WO-A1-92/00381
- WO-A1-98/03639
- DIALOG INFORMATION SERVICES, File 155, MEDLINE, Dialog Accession No. 08717474, Medline Accession No. 96266169, XP002940772, FIEROBE H.P. et al., "Mutational Modulation of Substrate Bond-Type Specificity and Thermostability of Glucoamylase from Aspergillus Awamori by Replacement with Short Homologue Active Site Sequences and Thiol/Disulfide Engineering"; & BIOCHEMISTRY, (UNITED STATES) 2 Jul. 1996, 35(26), pages 8696-8704.
- DIALOG INFORMATION SERVICES, File 155, MEDLINE, Dialog Accession No. 08501450, Medline Accession No. 96081441, XP002940773, CHEN H.M. et al., "Identification and Elimination by Site-Directed Mutagenesis of Thermoabile Aspartyl Bonds in Aspergillus Wawmori Glucoamylase"; & PROTEIN ENG., (ENGLAND) Jun. 1995, 8(6), pages 575-582.

## Description

### FIELD OF THE INVENTION

The present invention relates to novel glucoamylase variants (mutants) of parent AMG, in particular with improved thermal stability suitable for, *e.g*., starch conversion, *e.g*., for producing glucose from starch. More specifically, the present invention relates to glucoamylase enzyme variants and the use of such variant enzymes.

### BACKGROUND OF THE INVENTION

Glucoamylase (1,4-α-D-glucan glucohydrolase, EC 3.2.1.3) is an enzyme which catalyzes the release of D-glucose from the non-reducing ends of starch or related oligo- and polysaccharide molecules. Glucoamylases are produced by several filamentous fungi and yeasts, with those from *Aspergillus* being commercially most important.

Commercially, the glucoamylase enzyme is used to convert corn starch which is already partially hydrolyzed by an α-amylase to glucose. The glucose is further converted by glucose isomerase to a mixture composed almost equally of glucose and fructose. This mixture, or the mixture further enriched with fructose, is the commonly used high fructose corn syrup commercialized throughout the world. This syrup is the world's largest tonnage product produced by an enzymatic process. The three enzymes involved in the conversion of starch to fructose are among the most important industrial enzymes produced.

One of the main problems that exist with regard to the commercial use of glucoamylase in the production of high fructose corn syrup is the relatively low thermal stability of glucoamylase. Glucoamylase is not as thermally stable as α-amylase or glucose isomerase and it is most active and stable at lower pH's than either α-amylase or glucose isomerase. Accordingly, it must be used in a separate vessel at a lower temperature and pH.

Glucoamylase from *Aspergillus niger* has a catalytic (aa 1-440) and a starch binding domain (aa 509-616) separated by a long and highly *O*-glycosylated linker (Svensson et al. (1983), Carlsberg Res. Commun. 48, 529-544, 1983 and (1986), Eur. J. Biochem. 154, 497-502). The catalytic domain (aa 1-471) of glucoamylase from *A. awamori* var. *X100* adopt an (α/α)₆-fold in which six conserved α→α loop segments connect the outer and inner barrels (Aleshin et al. (1992), J. Biol.Chem. 267, 19291-19298). Crystal structures of glucoamylase in complex with 1-deoxynojirimycin (Harris et al. (1993), Biochemistry, 32, 1618-1626) and the pseudotetrasaccharide inhibitors acarbose and D-gluco-dihydroacarbose (Aleshin et al. (1996), Biochemistry 35, 8319-8328) furthermore are compatible with glutamic acids 179 and 400 acting as general acid and base, respectively. The crucial role of these residues during catalysis have also been studied using protein engineering (Sierks et al. (1990), Protein Engng. 3, 193-198; Frandsen et al. (1994), Biochemistry, 33, 13808-13816). Glucoamylase-carbohydrate interactions at four glycosyl residue binding subsites, -1, +1, +2, and +3 are highlighted in glucoamylase-complex structures (Aleshin et al. (1996), Biochemistry 35, 8319-8328) and residues important for binding and catalysis have been extensively investigated using site-directed mutants coupled with kinetic analysis (Sierks et al. (1989), Protein Engng. 2, 621-625; Sierks et al. (1990), Protein Engng. 3, 193-198; Berland et al. (1995), Biochemistry, 34, 10153-10161; Frandsen et al. (1995), Biochemistry, 34, 10162-10169.

Different substitutions in A. *niger* glucoamylase to enhance the thermal stability have been described: i) substitution of α-helical glycines: G137A and G139A (Chen et al. (1996), Prot. Engng. 9, 499-505); ii) elimination of the fragile Asp-X peptide bonds, D257E and D293E/Q (Chen et al. (1995), Prot. Engng. 8, 575-582); prevention of deamidation in N182 (Chen et al. (1994), Biochem. J. 301, 275-281); iv) engineering of additional disulphide bond, A246C (Fierobe et al. (1996), Biochemistry, 35, 8698-8704; and v) introduction of Pro residues in position A435 and S436 (Li et al. (1997), Protein Engng. 10, 1199-1204. Furthermore Clark Ford presented a paper on Oct 17, 1997, ENZYME ENGINEERING 14, Beijing/China Oct 12-17, 97, Abstract number: Abstract book p. 0-61. The abstract suggests mutations in positions G137A, N20C/A27C, and S30P in a (not disclosed) *Aspergillus awamori* glucoamylase to improve the thermal stability.

Additional information concerning glucoamylase can be found on an Internet homepage (http:/www.public.iastate.edu/~pedro/glase/glase.html)

"Glucoamylase WWW page" (Last changed 97/10/08) by Pedro M. Coutinho discloses informations concerning glucoamylases, including glucoamylases derivable from *Aspergillus* strains. Chemical and site-directed modifications in the *Aspergillus niger* glucoamylase are listed.

### BRIEF DISCLOSURE OF THE INVENTION

The object of the present invention is to provide improved glucoamylase variants with improved thermostablility suitable for use in, e. g. , the saccharification step in starch conversion processes.

The term "a glucoamylase variant with improved thermostability" means in the context of the present invention a glucoamylase variant which has a higher T_{1/2} (half-time) than the corresponding parent glucoamylase. The determination of T1/2 (Method I and Method II) is described below in the "Materials & Methods" section. The specific activity is determined as k_{cat} or AGU/mg (measured as described below in the "Materials & Methods" section).

··· The inventors of the present invention have provided a number of improved variants of a parent glucoamylase with improved thermostability comparison to the parent corresponding enzyme. The improved thermal stability is obtained by substituting selected positions in a parent glucoamylase. This will be described in details below.

### Nomenclature

In the present description and claims, the conventional one-letter and three-letter codes for amino acid residues are used. For ease of reference, glucoamylase variants of the invention are described by use of the following nomenclature:
Original amino acid(s):position(s):substituted amino acid(s)

According to this nomenclature, for instance the substitution of alanine for asparagine in position 30 is shown as:
Ala30Asn or A30N
a deletion of alanine in the same position is shown as:
Ala30* or A30*
and insertion of an additional amino acid residue, such as lysine, is shown as:
Ala30AlaLys or A30AK

A deletion of a consecutive stretch of amino acid residues, such as amino acid residues 30-33, is indicated as (30-33)* or Δ(A30-N33) .

Where a specific glucoamylase contains a "deletion" in comparison with other glucoamylases and an insertion is made in such a position this is indicated as:
*36Asp or *36D
for insertion of an aspartic acid in position 36
Multiple mutations are separated by plus signs, i.e.:
Ala30Asp + Glu34Ser or A30N+E34S
representing mutations in positions 30 and 34 substituting alanine and glutamic acid for asparagine and serine, respectively. Multiple mutation may also be separated as follows, i.e., meaning the same as the plus sign:
Ala30Asp/Glu34Ser or A30N/E34S

When one or more alternative amino acid residues may be inserted in a given position it is indicated as
A30N,E or A30N/E, or A30N or A30E

Furthermore, when a position suitable for modification is identified herein without any specific modification being suggested, it is to be understood that any amino acid residue may be substituted for the amino acid residue present in the position.

Thus, for instance, when a modification of an alanine in position 30 is mentioned, but not specified, it is to be understood that the alanine may be deleted or substituted for any other amino acid, i.e., any one of:
R,N,D,A,C,Q,E,G,H,I,L,K,M,F,P,S,T,W,Y,V.

### BRIEF DESCRIPTION OF THE DRAWING

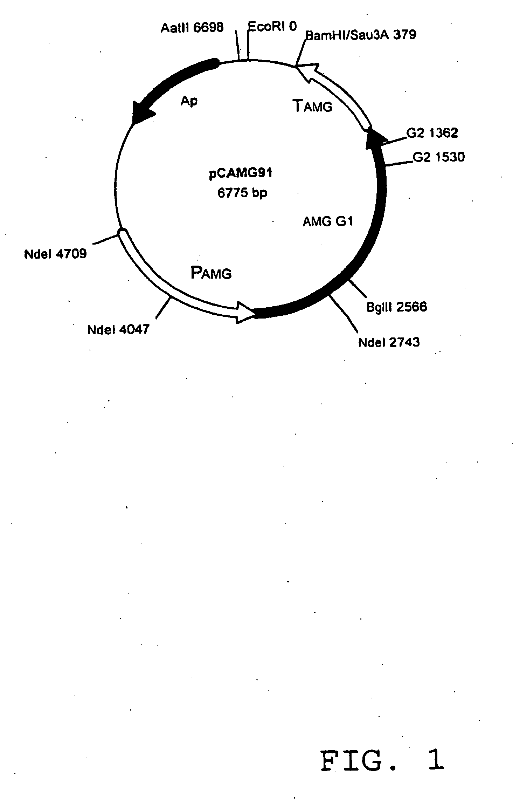

Figure 1 shows the plasmid pCAMG91 containing the *Aspergillus niger* G1 glucoamylase gene.

### DETAILED DISCLOSURE OF THE INVENTION

A goal of the work underlying the present invention was to improve the thermal stability of particular glucoamylases which are obtainable from fungal organisms, in particular strains of the *Aspergillus* genus and which themselves had been selected on the basis of their suitable properties in starch conversion or alcohol fermentation.

### Identifying positions and/or regions to be mutated to obtain Improved Thermostability

Molecular dynamics (MD) simulations indicate the mobility of the amino acids in the protein structure (see McCammon, JA and Harvey, SC., (1987), "Dynamics of proteins and nucleic acids". Cambridge University Press.). Such protein dynamics are often compared to the crystallographic B-factors (see Stout, GH and Jensen, LH, (1989), "X-ray structure determination", Wiley). By running the MD simulation at different protonation states of the titrate able residues, the pH related mobility of residues are simulated. Regions having the highest mobility or flexibility (here isotropic fluctuations) are selected for random mutagenesis. It is here understood that the high mobility found in certain areas of the protein, can be thermally improved by substituting residues in these residues. The substitutions are directed against residues that will change the dynamic behaviour of the residues to e.g. bigger side-chains and/or residues which have capability of forming improved contacts to residues in the near environment. The AMG from *Aspergillus niger* was used for the MD simulation. How to carry out MD simulation is described in the Materials & Methods" section below.)

Regions found by Molecular dynamics (MD) simulations to be suitable for mutation when wanting to obtain improved thermal stability are the following:
Region: 1-18,
Region: 19-35,
Region: 73-80,
Region: 200-212,
Region: 234-246,
Region: 334-341,
Region: 353-374,
Region: 407-411,
Region: 445-470,

Regions found to be of interest for increasing the thermostability are the regions in proximity to the active site. Regions positioned in between the α-helixes, and which may include positions on each side of the N- and C-terminal of the α-helixes, at the substrate binding site is of importance for the activity of the enzyme. These regions constitute the following regions:
Region : 40-62,
Region : 93-127,
Region : 170-184,
Region : 234-246,
Region : 287-319,
Region : 388-414.

*Rhizopus, Talaromyces,* such as *Talaromyces emersonii* (dislosed in WO 99/28448), and *Thielavia* have high specific activity towards maltodextrins, including maltose and maltohepatose. Therefore, regions being of special interest regarding (transferring) increased specific activity are:
Region: 200-212,
Region: 287-300,
Region: 305-319.

The present inventors have found that it is in fact possible to improve the thermal stability of a parent glucoamylase by modification of one or more amino acid residues of the amino acid sequence of the parent glucoamylase. The present invention is based on this finding.

Accordingly, in a first aspect the present invention relates to an improved variant of a parent glucoamylase comprising one or more mutations in the regions and positions described further below.

### Parent Glucoamylases

Parent glucoamylase contemplated according to the present invention include fungal glucoamylases, in particular fungal glucoamylases obtainable from an *Aspergillus* strain, such as an *Aspergillus niger* or *Aspergillus awamori* glucoamylases and variants or mutants thereof, homologous glucoamylases, and further glucoamylases being structurally and/or functionally similar to SEQ ID NO: 2. Specifically contemplated are the *Aspergillus niger* glucoamylases G1 and G2 disclosed in Boel et al. (1984), "Glucoamylases G1 and G2 from Aspergillus niger are synthesized from two different but closely related mRNAs", EMBO J. 3 (5), p. 1097-1102,. The G2 glucoamylase is disclosed in SEQ ID NO: 2. The G1 glucoamylase is disclosed in SEQ ID NO: 13 . Another AMG backbone contemplated is *Talaromyces emersonii* , especially *Talaromyces emersonii* DSM disclosed in WO 99/28448 (Novo Nordisk).

### Commercially Available Parent Glucoamylases

Commercially available parent glucoamylases include AMG from Novo Nordisk, and also glucoamylase from the companies Genencor, Inc. USA, and Gist-Brocades, Delft, The Netherlands.

### Glucoamylase Variants.

The invention relates to a variant of a parent glucoamylase as defined in the appended claims 1 to 3.

Also provided by the invention is a method for improving the thermostability of a parent glucoamylase, as defined in the appended claim 25.

### Homology (identity)

The homology referred to above of the parent glucoamylase is determined as the degree of identity between two protein sequences indicating a derivation of the first sequence from the second. The homology may suitably be determined by means of computer programs known in the art such as GAP provided in the GCG program package (Program Manual for the Wisconsin Package, Version 8, August 1994, Genetics Computer Group, 575 Science Drive, Madison, Wisconsin, USA 53711) (Needleman, S.B. and Wunsch, C.D., (1970), Journal of Molecular Biology, 48, p. 443-453). Using Gap with the following settings for polypeptide sequence comparison: Gap creation penalty of 3.0 and Gap extension penalty of 0.1, the mature part of a polypeptide encoded by an analogous DNA sequence of the invention exhibits a degree of identity preferably of at least 60%, such as 70%, at least 80%, at least 90%, more preferably at least 95%, more preferably at least 97%, and most preferably at least 99% with the mature part of the amino acid sequence shown in SEQ ID NO: 2.

Preferably, the parent glucoamylase comprise the amino acid sequences of SEQ ID NO: 2; or allelic variants thereof; or fragments thereof that has glucoamylase activity.

A fragment of SEQ ID NO: 2 is a polypeptide which have one or more amino acids deleted from the amino and/or carboxyl terminus of this amino acid sequence. For instance, the AMG G2 (SEQ ID NO: 2) is a fragment of the *Aspergillus niger* G1 glucoamylase (Boel et al. (1984), EMBO J. 3 (5), p. 1097-1102) having glucoamylase activity. An allelic variant denotes any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

The amino acid sequences of homologous parent glucoamylases may differ from the amino acid sequence of SEQ ID NO: 2 by an insertion or deletion of one or more amino acid residues and/or the substitution of one or more amino acid residues by different amino acid residues. Preferably, amino acid changes are of a minor nature, that is conservative amino acid substitutions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a poly-histidine tract, an antigenic epitope or a binding domain.

In another embodiment, the isolated parent glucoamylase is encoded by a nucleic acid sequence which hybridises under very low stringency conditions, preferably low stringency conditions, more preferably medium stringency conditions, more preferably medium-high stringency conditions, even more preferably high stringency conditions, and most preferably very high stringency conditions with a nucleic acid probe which hybridises under the same conditions with (i) the nucleic acid sequence of SEQ ID NO : 1, (ii) the cDNA sequence of SEQ ID NO:1, (iii) a sub-sequence of (i) or (ii), or (iv) a complementary strand of (i), (ii), or (iii) (J. Sambrook, E.F. Fritsch, and T. Maniatus, 1989, Molecular Cloning, A Laboratory Manual, 2d edition, Cold Spring Harbor, New York). The sub-sequence of SEQ ID NO: 1 may be at least 100 nucleotides or preferably at least 200 nucleotides. Moreover, the sub-sequence may encode a polypeptide fragment which has glucoamylase activity. The parent polypeptides may also be allelic variants or fragments of the polypeptides that have glucoamylase activity.

The nucleic acid sequence of SEQ ID NO: 1 or a subsequence thereof, as well as the amino acid sequence of SEQ ID NO: 2, or a fragment thereof, may be used to design a nucleic acid probe to identify and clone DNA encoding polypeptides having glucoamylase activity, from strains of different genera or species according to methods well known in the art. In particular, such probes can be used for hybridization with the genomic or cDNA of the genus or species of interest, following standard Southern blotting procedures, in order to identify and isolate the corresponding gene therein. Such probes can be considerably shorter than the entire sequence, but should be at least 15, preferably at least 25, and more preferably at least 35 nucleotides in length. Longer probes can also be used. Both DNA and RNA probes can be used. The probes are typically labeled for detecting the corresponding gene (for example, with ³²P, ³H, ³⁵S, biotin, or avidin). Such probes are encompassed by the present invention.

Thus, a genomic DNA or cDNA library prepared from such other organisms may be screened for DNA which hybridizes with the probes described above and which encodes a polypeptide having glucoamylase. Genomic or other DNA from such other organisms may be separated by agarose or polyacrylamide gel electrophoresis, or other separation techniques. DNA from the libraries or the separated DNA may be transferred to and immobilised on nitrocellulose or other suitable carrier material. In order to identify a clone or DNA which is homologous with SEQ ID NO: 1, or sub-sequences thereof, the carrier material is used in a Southern blot. For purposes of the present invention, hybridisation indicates that the nucleic acid sequence hybridises to a nucleic acid probe corresponding to the nucleic acid sequence shown in SEQ ID NO: 1 its complementary strand, or a sub-sequence thereof, under very low to very high stringency conditions. Molecules to which the nucleic acid probe hybridises under these conditions are detected using X-ray film.

For long probes of at least 100 nucleotides in length, the carrier material is finally washed three times each for 15 minutes using 2 x SSC, 0.2% SDS preferably at least at 45°C (very low stringency), more preferably at least at 50°C (low stringency), more preferably at least at 55°C (medium stringency), more preferably at least at 60°C (medium-high stringency), even more preferably at least at 65°C (high stringency), and most preferably at least at 70°C (very high stringency).

For short probes which are about 15 nucleotides to about 70 nucleotides in length, stringency conditions are defined as prehybridization, hybridisation, and washing post-hybridization at 5°C to 10°C below the calculated Tₘ using the calculation according to Bolton and McCarthy (1962, Proceedings of the National Academy of Sciences USA 48:1390) in 0.9 M NaCl, 0.09 M Tris-HCl pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per ml following standard Southern blotting procedures.

For short probes which are about 15 nucleotides to about 70 nucleotides in length, the carrier material is washed once in 6X SCC plus 0.1% SDS for 15 minutes and twice each for 15 minutes using 6x SSC at 5°C to 10°C below the calculated Tₘ.

The present invention also relates to isolated nucleic acid sequences produced by (a) hybridising a DNA under very low, low, medium, medium-high, high, or very high stringency conditions with the sequence of SEQ ID NO:1, or its complementary strand, or a sub-sequence thereof; and (b) isolating the nucleic acid sequence. The sub-sequence is preferably a sequence of at least 100 nucleotides such as a sequence which encodes a polypeptide fragment which has glucoamylase activity.

Contemplated parent glucoamylases have at least 20%, preferably at least 40%, more preferably at least 60%, even more preferably at least 80%, even more preferably at least 90%, and most preferably at least 100% of the glucoamylase activity of the mature polypeptide of SEQ ID NO: 2.

In a preferred embodiment the variant of the invention has improved thermal stability, preferably within the temperature interval from about 60-80°C, preferably 63-75°C, preferably at a pH of 4-5, in particular 4.2-4.7, using maltodextrin as the substrate.

In another preferred embodiment a variant of the invention is used for, e.g., alcohol fermentation.

In a preferred embodiment the parent glucoamylase is the *Aspergillus niger* G1 glucoamylase (Boel et al. (1984), EMBO J. 3 (5), p. 1097-1102. The parent glucoamylase may be a truncated glucoamylase, e.g., the AMG G2 glucoamylase.

### Cloning a DNA Sequence Encoding A Parent Glucoamylase

The DNA sequence encoding a parent glucoamylase may be isolated from any cell or microorganism producing the glucoamylase in question, using various methods well known in the art. First, a genomic DNA and/or cDNA library should be constructed using chromosomal DNA or messenger RNA from the organism that produces the glucoamylase to be studied. Then, if the amino acid sequence of the glucoamylase is known, labeled oligonucleotide probes may be synthesized and used to identify glucoamylase-encoding clones from a genomic library prepared from the organism in question. Alternatively, a labelled oligonucleotide probe containing sequences homologous to another known glucoamylase gene could be used as a probe to identify glucoamylase-encoding clones, using hybridization and washing conditions of very low to very high stringency. This is described above.

Yet another method for identifying glucoamylase-encoding clones would involve inserting fragments of genomic DNA into an expression vector, such as a plasmid, transforming glucoamylase-negative bacteria with the resulting genomic DNA library, and then plating the transformed bacteria onto agar containing a substrate for glucoamylase *(i.e.,* maltose), thereby allowing clones expressing the glucoamylase to be identified.

Alternatively, the DNA sequence encoding the enzyme may be prepared synthetically by established standard methods, e.g. the phosphoroamidite method described S.L. Beaucage and M.H. Caruthers, (1981), Tetrahedron Letters 22, p. 1859-1869, or the method described by Matthes et al., (1984), EMBO J. 3, p. 801-805. In the phosphoroamidite method, oligonucleotides are synthesized, e.g., in an automatic DNA synthesizer, purified, annealed, ligated and cloned in appropriate vectors.

Finally, the DNA sequence may be of mixed genomic and synthetic origin, mixed synthetic and cDNA origin or mixed genomic and cDNA origin, prepared by ligating fragments of synthetic, genomic or cDNA origin (as appropriate, the fragments corresponding to various parts of the entire DNA sequence), in accordance with standard techniques. The DNA sequence may also be prepared by polymerase chain reaction (PCR) using specific primers, for instance as described in US 4,683,202 or R.K. Saiki et al., (1988), Science 239, 1988, pp. 487-491.

### Site-directed mutagenesis

Once a glucoamylase-encoding DNA sequence has been isolated, and desirable sites for mutation identified, mutations may be introduced using synthetic oligonucleotides. These oligonucleotides contain nucleotide sequences flanking the desired mutation sites. In a specific method, a single-stranded gap of DNA, the glucoamylase-encoding sequence, is created in a vector carrying the glucoamylase gene. Then the synthetic nucleotide, bearing the desired mutation, is annealed to a homologous portion of the single-stranded DNA. The remaining gap is then filled in with DNA polymerase I (Klenow fragment) and the construct is ligated using T4 ligase. A specific example of this method is described in Morinaga et al., (1984), Biotechnology 2, p. 646-639. US 4,760,025 discloses the introduction of oligonucleotides encoding multiple mutations by performing minor alterations of the cassette. However, an even greater variety of mutations can be introduced at any one time by the Morinaga method, because a multitude of oligonucleotides, of various lengths, can be introduced.

Another method for introducing mutations into glucoamylase-encoding DNA sequences is described in Nelson and Long, (1989), Analytical Biochemistry 180, p. 147-151. It involves the 3-step generation of a PCR fragment containing the desired mutation introduced by using a chemically synthesized DNA strand as one of the primers in the PCR reactions. From the PCR-generated fragment, a DNA fragment carrying the mutation may be isolated by cleavage with restriction endonucleases and reinserted into an expression plasmid.

Further, Sierks. et al., (1989), Protein Eng., 2, 621-625; Sierks et al., (1990), Protein Eng. vol. 3, 193-198; also describes site-directed mutagenesis in an *Aspergillus* glucoamylase.

### Random Mutagenesis

Random mutagenesis is suitably performed either as localized or region-specific random mutagenesis in at least three parts of the gene translating to the amino acid sequence shown in question, or within the whole gene.

The random mutagenesis of a DNA sequence encoding a parent glucoamylase may be conveniently performed by use of any method known in the art.

In relation to the above, a further aspect of the present invention relates to a method for generating a variant of a parent glucoamylase, wherein the variant exhibits increased thermal stability relative to the parent, the method comprising:
(a) subjecting a DNA sequence encoding the parent glucoamylase to random mutagenesis,
(b) expressing the mutated DNA sequence obtained in step (a) in a host cell, and
(c) screening for host cells expressing a glucoamylase variant which has an altered property (i.e. thermal stability) relative to the parent glucoamylase.

Step (a) of the above method of the invention is preferably performed using doped primers, as described in the working examples herein (vide *infra).*

For instance, the random mutagenesis may be performed by use of a suitable physical or chemical mutagenizing agent, by use of a suitable oligonucleotide, or by subjecting the DNA sequence to PCR generated mutagenesis. Furthermore, the random mutagenesis may be performed by use of any combination of these mutagenizing agents. The mutagenizing agent may, e.g., be one which induces transitions, transversions, inversions, scrambling, deletions, and/or insertions.

Examples of a physical or chemical mutagenizing agent suitable for the present purpose include ultraviolet (UV) ir-radiation, hydroxylamine, N-methyl-N'-nitro-N-nitrosoguanidine (MNNG), O-methyl hydroxylamine, nitrous acid, ethyl methane sulphonate (EMS), sodium bisulphite, formic acid, and nucleotide analogues. When such agents are used, the mutagenesis is typically performed by incubating the DNA sequence encoding the parent enzyme to be mutagenized in the presence of the mutagenizing agent of choice under suitable conditions for the mutagenesis to take place, and selecting for mutated DNA having the desired properties.

When the mutagenesis is performed by the use of an oligonucleotide, the oligonucleotide may be doped or spiked with the three non-parent nucleotides during the synthesis of the oligonucleotide at the positions which are to be changed. The doping or spiking may be done so that codons for unwanted amino acids are avoided. The doped or spiked oligonucleotide can be incorporated into the DNA encoding the glucoamylase enzyme by any published technique, using, *e.g.,* PCR, LCR or any DNA polymerase and ligase as deemed appropriate.

Preferably, the doping is carried out using "constant random doping", in which the percentage of wild-type and mutation in each position is predefined. Furthermore, the doping may be directed toward a preference for the introduction of certain nucleotides, and thereby a preference for the introduction of one or more specific amino acid residues. The doping may be made, e.g., so as to allow for the introduction of 90% wild type and 10% mutations in each position. An additional consideration in the choice of a doping scheme is based on genetic as well as protein-structural constraints. The doping scheme may be made by using the DOPE program which, *inter alia,* ensures that introduction of stop codons is avoided.

When PCR-generated mutagenesis is used, either a chemically treated or non-treated gene encoding a parent glucoamylase is subjected to PCR under conditions that increase the misincorporation of nucleotides (Deshler 1992; Leung et al., Technique, Vol.1, 1989, pp. 11-15).

A mutator strain of E. *coli* (Fowler et al., Molec. Gen. Genet., 133, 1974, pp. 179-191), *S. cereviseae* or any other microbial organism may be used for the random mutagenesis of the DNA encoding the glucoamylase by, e.g., transforming a plasmid containing the parent glycosylase into the mutator strain, growing the mutator strain with the plasmid and isolating the mutated plasmid from the mutator strain. The mu-ta-ted plasmid may be subsequently transformed into the expression organism.

The DNA sequence to be mutagenized may be conveniently present in a genomic or cDNA library prepared from an organism expressing the parent glucoamylase. Alternatively, the DNA sequence may be present on a suitable vector such as a plasmid or a bacteriophage, which as such may be incubated with or other-wise exposed to the mutagenising agent. The DNA to be mutagenized may also be present in a host cell either by being integrated in the genome of said cell or by being present on a vector harboured in the cell. Finally, the DNA to be mutagenized may be in isolated form. It will be understood that the DNA sequence to be subjected to random mutagenesis is preferably a cDNA or a genomic DNA sequence.

In some cases it may be convenient to amplify the mutated DNA sequence prior to performing the expression step b) or the screening step c). Such amplification may be performed in accordance with methods known in the art, the presently preferred method being PCR-generated amplification using oligonucleotide primers prepared on the basis of the DNA or amino acid sequence of the parent enzyme.

Subsequent to the incubation with or exposure to the mutagenising agent, the mutated DNA is expressed by culturing a suitable host cell carrying the DNA sequence under conditions allowing expression to take place. The host cell used for this purpose may be one which has been transformed with the mutated DNA sequence, optionally present on a vector, or one which carried the DNA sequence encoding the parent enzyme during the mutagenesis treatment. Examples of suitable host cells are the following: gram positive bacteria such as *Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus, Bacillus megaterium, Bacillus thuringiensis, Streptomyces lividans* or *Streptomyces murinus;* and gram-negative bacteria such as *E. coli.*

The mutated DNA sequence may further comprise a DNA sequence encoding functions permitting expression of the mutated DNA sequence.

### Localized random mutagenesis

The random mutagenesis may be advantageously localized to a part of the parent glucoamylase in question. This may, *e.g.,* be advantageous when certain regions of the enzyme have been identified to be of particular importance for a given property of the enzyme, and when modified are expected to result in a variant having improved properties. Such regions may normally be identified when the tertiary structure of the parent enzyme has been elucidated and related to the function of the enzyme.

The localized, or region-specific, random mutagenesis is conveniently performed by use of PCR generated mutagenesis techniques as described above or any other suitable technique known in the art. Alternatively, the DNA sequence encoding the part of the DNA sequence to be modified may be isolated, e.g., by insertion into a suitable vector, and said part may be subsequently subjected to mutagenesis by use of any of the mutagenesis methods discussed above.

Alternative methods for providing variants of the invention include gene shuffling e.g. as described in WO 95/22625 (from Affymax Technologies N.V.) or in WO 96/00343 (from Novo Nordisk A/S).

### Expression Of Glucoamylase Variants

According to the invention, a DNA sequence encoding the variant produced by methods described above, or by any alternative methods known in the art, can be expressed, in enzyme form, using an expression vector which typically includes control sequences encoding a promoter, operator, ribosome binding site, translation initiation signal, and, optionally, a repressor gene or various activator genes.

### Expression vector

The recombinant expression vector carrying the DNA sequence encoding a glucoamylase variant of the invention may be any vector which may conveniently be subjected to recombinant DNA procedures, and the choice of vector will often depend on the host cell into which it is to be introduced. The vector may be one which, when introduced into a host cell, is integrated into the host cell genome and replicated together with the chromosome(s) into which it has been integrated. Examples of suitable expression vectors include pMT838.

### Promoter

In the vector, the DNA sequence should be operably connected to a suitable promoter sequence. The promoter may be any DNA sequence which shows transcriptional activity in the host cell of choice and may be derived from genes encoding proteins either homologous or heterologous to the host cell.

Examples of suitable promoters for directing the transcription of the DNA sequence encoding a glucoamylase variant of the invention, especially in a bacterial host, are the promoter of the *lac* operon of *E.coli,* the *Streptomyces coelicolor* agarase gene *dag*A promoters, the promoters of the *Bacillus licheniformis α-*amylase gene *(amyL),* the promoters of the *Bacillus stearothermophilus* maltogenic amylase gene *(amyM),* the promoters of the *Bacillus amyloliquefaciens* α-amylase *(amyQ) ,* the promoters of the *Bacillus subtilis* xy1A and xylB genes etc. For transcription in a fungal host, examples of useful promoters are those derived from the gene encoding A. *oryzae* TAKA amylase, the TPI (triose phosphate isomerase) promoter from S. *cerevisiae* (Alber et al. (1982), J. Mol. Appl. Genet 1, p. 419-434, Rhizo*mucor miehei* aspartic proteinase, *A. niger* neutral α-amylase, A. *niger* acid stable α-amylase, *A. niger* glucoamylase, *Rhizomucor miehei* lipase, *A. oryzae* alkaline protease, A. *oryzae* triose phosphate isomerase or *A. nidulans* acetamidase.

### Expression vector

The expression vector of the invention may also comprise a suitable transcription terminator and, in eukaryotes, polyadenylation sequences operably connected to the DNA sequence encoding the α-amylase variant of the invention. Termination and polyadenylation sequences may suitably be derived from the same sources as the promoter.

The vector may further comprise a DNA sequence enabling the vector to replicate in the host cell in question. Examples of such sequences are the origins of replication of plasmids pUC19, pACYC177, pUB110, pE194, pAMB1 and pIJ702.

The vector may also comprise a selectable marker, e.g. a gene the product of which complements a defect in the host cell, such as the *dal* genes from *B. subtilis* or *B. licheniformis,* or one which confers antibiotic resistance such as ampicillin, kanamycin, chloramphenicol or tetracyclin resistance. Furthermore, the vector may comprise *Aspergillus* selection markers such as amdS, argB, niaD and sC, a marker giving rise to hygromycin resistance, or the selection may be accomplished by co-transformation, e.g. as described in WO 91/17243.

The procedures used to ligate the DNA construct of the invention encoding a glucoamylase variant, the promoter, terminator and other elements, respectively, and to insert them into suitable vectors containing the information necessary for replication, are well known to persons skilled in the art (cf., for instance, Sambrook et al., Molecular Cloning: A Laboratory Manual, 2nd Ed., Cold Spring Harbor, 1989).

### Host Cells

The cell of the invention, either comprising a DNA construct or an expression vector of the invention as defined above, is advantageously used as a host cell in the recombinant production of a glucoamylase variant of the invention. The cell may be transformed with the DNA construct of the invention encoding the variant, conveniently by integrating the DNA construct (in one or more copies) in the host chromosome. This integration is generally considered to be an advantage as the DNA sequence is more likely to be stably maintained in the cell. Integration of the DNA constructs into the host chromosome may be performed according to conventional methods, e.g. by homologous or heterologous recombination. Alternatively, the cell may be transformed with an expression vector as described above in connection with the different types of host cells.

The cell of the invention may be a cell of a higher organism such as a mammal or an insect, but is preferably a microbial cell, e.g., a bacterial or a fungal (including yeast) cell.

Examples of suitable bacteria are Gram positive bacteria such as *Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus, Bacillus megaterium, Bacillus thuringiensis,* or *Streptomyces lividans* or *Streptomyces murinus,* or gramnegative bacteria such as *E.coli.* The transformation of the bacteria may, for instance, be effected by protoplast transformation or by using competent cells in a manner known *per se.*

The yeast organism may favorably be selected from a species of *Saccharomyces* or *Schizosaccharomyces, e.g. Saccharomyces cerevisiae.*

The host cell may also be a filamentous fungus *e.g.* a strain belonging to a species of *Aspergillus,* most preferably *Aspergillus oryzae* or *Aspergillus* niger, or a strain of *Fusarium,* such as a strain of *Fusarium oxysporium, Fusarium graminearum* (in the perfect state named *Gribberella zeae,* previously *Sphaeria zeae, synonym with Gibberella roseum and Gibberella roseum* f. sp. *cerealis),* or *Fusarium sulphureum* (in the prefect state named *Gibberella puricaris,* synonym with *Fusarium trichothecioides, Fusarium bactridioides, Fusarium sambucium, Fusarium roseum,* and *Fusarium roseum var. graminearum), Fusarium cerealis* (synonym with *Fusarium crokkwellnse), or Fusarium venenatum.*

In a preferred embodiment of the invention the host cell is a protease deficient or protease minus strain.

This may for instance be the protease deficient strain *Aspergillus oryzae* JaL 125 having the alkaline protease gene named "alp" deleted. This strain is described in WO 97/35956 (Novo Nordisk).

Filamentous fungi cells may be transformed by a process involving protoplast formation and transformation of the protoplasts followed by regeneration of the cell wall in a manner known per se. The use of *Aspergillus* as a host microorganism is described in EP 238 023 (Novo Nordisk A/S),

### Method Of Producing A Glucoamylase Variant

In a yet further aspect, the present invention relates to a method of producing a glucoamylase variant of the invention, which method comprises cultivating a host cell under conditions conducive to the production of the variant and recovering the variant from the cells and/or culture medium.

The medium used to cultivate the cells may be any conventional medium suitable for growing the host cell in question and obtaining expression of the glucoamylase variant of the invention. Suitable media are available from commercial suppliers or may be prepared according to published recipes (e.g. as described in catalogues of the American Type Culture Collection).

The glucoamylase variant secreted from the host cells may conveniently be recovered from the culture medium by well-known procedures, including separating the cells from the medium by centrifugation or filtration, and precipitating proteinaceous components of the medium by means of a salt such as ammonium sulphate, followed by the use of chromatographic procedures such as ion exchange chromatography, affinity chromatography, or the like.

### Starch Conversion

The present invention provides a method of using glucoamylase variants of the invention for producing glucose and the like from starch. Generally, the method includes the steps of partially hydrolyzing precursor starch in the presence of α-amylase and then further hydrolyzing the release of D-glucose from the non-reducing ends of the starch or related oligo- and polysaccharide molecules in the presence of glucoamylase by cleaving α-(1 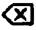 4) and α-(1 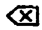 6) glucosidic bonds.

The partial hydrolysis of the precursor starch utilizing α-amylase provides an initial breakdown of the starch molecules by hydrolyzing internal α-(1 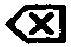 4)-linkages. In commercial applications, the initial hydrolysis using α-amylase is run at a temperature of approximately 105°C. A very high starch concentration is processed, usually 30% to 40% solids. The initial hydrolysis is usually carried out for five minutes at this elevated temperature. The partially hydrolyzed starch can then be transferred to a second tank and incubated for approximately 1-2 hour at a temperature of 85° to 98°C to derive a dextrose equivalent (D.E.) of 10 to 15.

The step of further hydrolyzing the release of D-glucose from the non-reducing ends of the starch or related oligo- and polysaccharides molecules in the presence of glucoamylase is normally carried out in a separate tank at a reduced temperature between 30° and 62°C. Preferably the temperature of the substrate liquid is dropped to between 55° and 60°C. The pH of the solution is dropped from about 5.5 to 6.5 to a range between 3 and 5.5. Preferably, the pH of the solution is 4 to 4.5. The glucoamylase is added to the solution and the reaction is carried out for 24-72 hours, preferably 36-48 hours.

By using a thermostable glucoamylase variant of the invention saccharification processes may be carried out at a higher temperature than traditional batch saccharification processes. According to the invention saccharification may be carried out at temperatures in the range from above 60-80°C, preferably 63-75°C. This apply both for traditional batch processes (described above) and for continuous saccharification processes.

Actually, continuous saccharification processes including one or more membrane separation steps, i.e. filtration steps, must be carried out at temperatures of above 60°C to be able to maintain a reasonably high flux over the membrane or to minimize microbial contamination. Therefore, the thermostable variants of the invention provides the possibility of carrying out large scale continuous saccharification processes at a fair price and/or at a lower enzyme protein dosage within a period of time acceptable for industrial saccharification processes. According to the invention the saccharification time may even be shortened.

The activity of the glucoamylase variant (e.g. AMG variant) of the invention is generally substantially higher at temperatures between 60°C-80°C than at the traditionally used temperature between 30-60°C. Therefore, by increasing the temperature at which the glucoamylase operates the saccharification process may be carried out within a shorter period of time.

Further, by improving the thermal stability the T_{½} (half-time, as defined in the "Materials and Methods" section) is improved. As the thermal stability of the glucoamylase variants of the invention is improved a minor amount of glucoamylase need to be added to replace the glucoamylase being inactivated during the saccharification process. More glucoamylase is maintained active during saccharification process according to the present invention. Furthermore, the risk of microbial contamination is also reduced when carrying the saccharification process at temperature above 63°C.

The glucose yield from a typical saccharification trial with glucoamylase, acid amylase and pullulanase is 95.5-96.5%. The remaining carbohydrates typically consists of 1% maltose, 1.5-2% isomaltose and 1-1.5% higher oligosacharides. The disaccharides are produced since the glucoamylase at high concentrations of glucose and high dry-solid levels has a tendency to form reversion products.

A glucoamylase with an increased specific activity towards saccharides present in the solution after liquefaction and saccharides formed during saccharification would be an advantage as a reduced enzyme protein dosage or a shorter process time then could be used. In general, the glucoamylase has a preference for substrates consisting of longer saccharides compared to short chain saccharides and the specific activity towards e.g. maltoheptaose is therefore approximately 6 times higher than towards maltose. An increased specific activity towards short chain saccharides such as maltose (without reducing the activity towards oligosaccharides) would therefore also permit using a lower enzyme dosage and/or shorter process time.

Furthermore, a higher glucose yield can be obtained with a glucoamylase variant with an increased alpha-1,4 hydrolytic activity (if the alpha-1,6 activity is unchanged or even decreased), since a reduced amount of enzyme protein is being used, and alpha-1,6 reversion product formation therefore is decreased (less isomaltose).

The specific activity may be measured using the method described in the "Materials & Methods" section at 37°C or 60°C.

Example of saccharification process wherein the glucoamylase variants of the invention may be used include the processes described in JP 3-224493; JP 1-191693;JP 62-272987; EP 452,238, and WO 99/27124.

In a further aspect the invention relates to a method of saccharifying a liquefied starch solution, comprising the steps
(i) a saccharification step during which step one or more enzymatic saccharification stages takes place, and the subsequent step of
(ii) one or more high temperature membrane separation steps
wherein the enzymatic saccharification is carried out using a thermostable glucoamylase variant of the invention.

The glucoamylase variant(s) of the invention may be used in the present inventive process in combination with an enzyme that hydrolyzes only α-(1 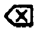 6)-glucosidic bonds in molecules with at least four glucosyl residues. Preferentially, the glucoamylase variant of the invention can be used in combination with pullulanase or isoamylase. The use of isoamylase and pullulanase for debranching, the molecular properties of the enzymes, and the potential use of the enzymes with glucoamylase is set forth in G.M.A. van Beynum et al., Starch Conversion Technology, Marcel Dekker, New York, 1985, 101-142.

In a further aspect the invention relates to the use of a glucoamylase variant of the invention in a starch conversion process.

Further, the glucoamylase variant of the invention may be used in a continuous starch conversion process including a continuous saccharification step.

The glucoamylase variants of the invention may also be used in immobilised form. This is suitable and often used for producing speciality syrups, such as maltose syrups, and further for the raffinate stream of oligosaccharides in connection with the production of fructose syrups.

The glucoamylase of the invention may also be used in a process for producing ethanol for fuel or beverage or may be used in a fermentation process for producing organic compounds, such as citric acid, ascorbic acid, lysine, glutamic acid.

### MATERIALS & METHODS

### MATERIALS:

### Enzymes:

AMG G1: *Aspergillus niger* glucoamylase G1 disclosed in Boel et al. (1984), EMBO J. 3 (5), 1097-1102, and SEQ ID NO: 13, available from Novo Nordisk.
AMG G2: Truncated *Aspergillus niger* glucoamylase G1 shown in SEQ ID NO: 2, available from Novo Nordisk)

### Solutions:

Buffer: 0.05M sodium acetate (6.8g in 1 1 milli-Q-water), pH 4.5
Stop solution: 0.4M NaOH
GOD-perid, 124036, Boehringer Mannheim

### Substrate:

Maltose: 29mM (1g maltose in 100ml 50mM sodium acetate, pH 4.5) (Sigma)
Maltoheptaose: 10 mM, 115 mg/10 ml (Sigma)

### Host cell:

*A. oryzae* JaL 125: *Aspergillus oryzae* IFO 4177 available from Institute for Fermention, Osaka; 17-25 Juso Hammachi 2-Chome Yodogawa-ku, Osaka, Japan, having the alkaline protease gene named "alp" (described by Murakami K et al., (1991), Agric. Biol. Chem. 55, p. 2807-2811) deleted by a one step gene replacement method (described by G. May in "Applied Molecular Genetics of Filamentous Fungi" (1992), p. 1-25. Eds. J. R. Kinghorn and G. Turner; Blackie Academic and Professional), using the *A. oryzae* pyrG gene as marker. Strain JaL 125 is further disclosed in WO 97/35956 (Novo Nordisk).

### Micro-organisms:

Strain: Saccharomyces cerevisiae YNG318: MATαleu2-Δ2 ura3-52 his4-539 pep4-Δ1[cir+]

### Plasmids:

pCAMG91: see Figure 1. Plasmid comprising the *Aspergillus niger* G1 glucoamylase (AMG G1). The construction of pCAMG91 is described in Boel et al. (1984), EMBO J. 3 (7) p.1581-1585.

pMT838: Plasmid encoding the truncated *Aspergillus niger* glucoamylase G2 (SEQ ID NO: 2).

pJS0026 (S. cerevisiae expression plasmid)(J.S.Okkels, (1996)"A URA3-promoter deletion in a pYES vector increases the expression level of a fungal lipase in Saccharomyces cerevisiae. Recombinant DNA Biotechnology III: The Integration of Biological and Engineering Sciences, vol. 782 of the Annals of the New York Academy of Sciences) More specifically, the expression plasmid pJSO37, is derived from pYES 2.0 by replacing the inducible GAL1- promoter of pYES 2.0 with the constitutively expressed TPI (triose phosphate isomerase)-promoter from Saccharomyces cerevisiae (Albert and Karwasaki, (1982), J. Mol. Appl Genet., 1, 419-434), and deleting a part of the URA3 promoter.

### METHODS:

### Transformation of Saccharomyces cerevisiae YNG318

The DNA fragments and the opened vectors are mixed and transformed into the yeast *Saccharomyces cerevisiae* YNG318 by standard methods.

### Determining Specific Activity As k_{cat} (sec.⁻¹).

750 microL substrate (1% maltose, 50 mM Sodium acetat, pH 4.3) is incubated 5 minutes at selected temperature, such as 37°C or 60°C.

50 microL enzyme diluted in sodium acetate is added. Aliquots of 100 microL are removed after 0, 3, 6, 9 and 12 minutes and transferred to 100 microL 0.4 M Sodium hydroxide to stop the reaction. A blank is included.
20 microL is transferred to a Micro titre plates and 200 microL GOD-Perid solution is added. Absorbance is measured at 650 nm after 30 minutes incubation at room temperature.
Glucose is used as standard and the specific activity is calculated as k_{cat} (sec.⁻¹).

### Determination Of AGU Activity and As AGU/mg

One Novo Amyloglucosidase Unit (AGU) is defined as the amount of enzyme which hydrolyzes 1 micromole maltose per minute at 37°C and pH 4.3. A detailed description of the analytical method (AEL-SM-0131) is available on request from Novo Nordisk .

The activity is determined as AGU/ml by a method modified after (AEL-SM-0131) using the Glucose GOD-Perid kit from Boehringer Mannheim, 124036. Standard: AMG-standard, batch 7-1195, 195 AGU/ml.

375 microL substrate (1% maltose in 50 mM Sodium acetate, pH 4.3) is incubated 5 minutes at 37°C. 25 microL enzyme diluted in sodium acetate is added. The reaction is stopped after 10 minutes by adding 100 microL 0.25 M NaOH. 20 microL is transferred to a 96 well microtitre plate and 200 microL GOD-Perid solution is added. After 30 minutes at room temperature, the absorbance is measured at 650 nm and the activity calculated in AGU/ml from the AMG-standard.

The specific activity in AGU/mg is then calculated from the activity (AGU/ml) divided with the protein concentration (mg/ml).

### Transformation Of Aspergillus oryzae (general procedure)

100 ml of YPD (Sherman et al., (1981), Methods in Yeast Genetics, Cold Spring Harbor Laboratory) are inoculated with spores of A. *oryzae* and incubated with shaking for about 24 hours. The mycelium is harvested by filtration through miracloth and washed with 200 ml of 0.6 M MgSO₄. The mycelium is suspended in 15 ml of 1.2 M MgSO₄ 10 mM NaH₂PO₄, pH 5.8. The suspension is cooled on ice and 1 ml of buffer containing 120 mg of Novozym^{™} 234 is added. After 5 min., 1 ml of 12 mg/ml BSA (Sigma type H25) is added and incubation with gentle agitation continued for 1.5-2.5 hours at 37C until a large number of protoplasts is visible in a sample inspected under the microscope.

The suspension is filtered through miracloth, the filtrate transferred to a sterile tube and overlayed with 5 ml of 0.6 M sorbitol, 100 mM Tris-HCl, pH 7.0. Centrifugation is performed for 15 min. at 1000 g and the protoplasts are collected from the top of the MgSO₄ cushion. 2 volumes of STC (1.2 M sorbitol, 10 mM Tris-HCl, pH 7.5, 10 mM CaCl₂) are added to the protoplast suspension and the mixture is centrifugated for 5 min. at 1000 g. The protoplast pellet is resuspended in 3 ml of STC and repelleted. This is repeated. Finally, the protoplasts are resuspended in 0.2-1 ml of STC.

100 µl of protoplast suspension are mixed with 5-25 µg of p3SR2 (an *A*. *nidulans* amdS gene carrying plasmid described in Hynes et al., Mol. and Cel. Biol., Vol. 3, No. 8, 1430-1439, Aug. 1983) in 10 µl of STC. The mixture is left at room temperature for 25 min. 0.2 ml of 60% PEG 4000 (BDH 29576), 10 mM CaCl₂ and 10 mM Tris-HCl, pH 7.5 is added and carefully mixed (twice) and finally 0.85 ml of the same solution are added and carefully mixed. The mixture is left at room temperature for 25 min., spun at 2.500 g for 15 min. and the pellet is resuspended in 2 ml of 1.2M sorbitol. After one more sedimentation the protoplasts are spread on minimal plates (Cove, (1966), Biochem. Biophys. Acta 113, 51-56) containing 1.0 M sucrose, pH 7.0, 10 mM acetamide as nitrogen source and 20 mM CsCl to inhibit background growth. After incubation for 4-7 days at 37°C spores are picked, suspended in sterile water and spread for single colonies. This procedure is repeated and spores of a single colony after the second re-isolation are stored as a defined transformant.

### Fed Batch Fermentation

Fed batch fermentation is performed in a medium comprising maltodextrin as a carbon source, urea as a nitrogen source and yeast extract. The fed batch fermentation is performed by inoculating a shake flask culture of *A. oryzae* host cells in question into a medium comprising 3.5% of the carbon source and 0.5% of the nitrogen source. After 24 hours of cultivation at pH 5.0 and 34°C the continuous supply of additional carbon and nitrogen sources are initiated. The carbon source is kept as the limiting factor and it is secured that oxygen is present in excess. The fed batch cultivation is continued for 4 days, after which the enzymes can be recovered by centrifugation, ultrafiltration, clear filtration and germ filtration.

### Purification

The culture broth is filtrated and added ammoniumsulphate (AMS) to a concentration of 1.7 M AMS and pH is adjusted to pH 5. Precipitated material is removed by centrifugation and the solution containing glucoamylase activity is applied on a Toyo Pearl Butyl column previously equilibrated in 1.7 M AMS, 20 mM sodium acetate, pH 5. Unbound material is washed out with the equilibration buffer. Bound proteins are eluted with 10 mM sodium acetate, pH 4.5 using a linear gradient from 1.7 - 0 M AMS over 10 column volumes. Glucoamylase containing fractions are collected and dialysed against 20 mM sodium acetate, pH 4.5. The solution was then applied on a Q sepharose column, previously equilibrated in 10 mM Piperazin, Sigma, pH 5.5. Unbound material is washed out with the equilibration buffer. Bound proteins are eluted with a linear gradient of 0-0.3 M Sodium chloride in 10 mM Piperazin, pH 5.5 over 10 column volumes. Glucoamylase containing fractions are collected and the purity was confirmed by SDS-PAGE.

### T_{1/2} (half-life) Method I

The thermal stability of variants is determined as T_{½} using the following method: 950 microliter 50 mM sodium acetate buffer (pH 4.3) (NaOAc) is incubated for 5 minutes at 68°C or 70°C. 50 microliter enzyme in buffer (4 AGU/ml) is added. 2 x 40 microliter samples are taken at 0, 5, 10, 20, 30 and 40 minutes and chilled on ice. The activity (AGU/ml) measured before incubation (0 minutes) is used as reference (100%). The decline in stability (in percent) is calculated as a function of the incubation time. The % residual glucoamylase activity is determined at different times. T_{½} is the period of time until which the % relative activity is decreased to 50%.

### T_{1/2} (half-life) (Method II)

The T_{½} is measured by incubating the enzyme (ca 0.2 AGU/ml) in question in 30% glucose, 50 mM Sodium acetate at pH 4.5 at the temperature in question (e.g., 70°C). Samples are withdrawn at set time intervals and chilled on ice and residual enzyme activity measured by the pNPG method (as described below).

The % residual glucoamylase activity is determined at different times. T_{1/2} is the period of time until which the % relative activity is decreased to 50%.

### Residual Enzyme Activity (pNPG method)

### pNPG reagent:

0.2 g pNPG (p-nitrophenylglucopyranoside) is dissolved in 0.1 M acetate buffer (pH 4.3) and made up to 100 ml.

### Borate solution:

3.8g Na₂B₄O₇ 10 H₂O is dissolved in Milli-Q water and made up to 100 ml.

25 microL samples are added 50 microL substrate and incubated 2 hr at 50°C. The reaction is stopped by adding 150 micoL ml borate solution. The optical density is measured at 405 nm, and the residual activity calculated.

### Construction Of pAMGY

The pAMGY vector was constructed as follows: The lipase gene in pJSO026 was replaced by the AMG gene, which was PCR amplified with the forward primer; FG2: 5'-CAT CCC CAG GAT CCT TAC TCA GCA ATG-3' and the reverse primer: RG2: 5'-CTC AAA CGA CTC ACC AGC CTC TAG AGT-3' using the template plasmid pLAC103 containing the AMG gene. The pJSO026 plasmid was digested with XbaI and SmaI at 37°C for 2 hours and the PCR amplicon was blunt ended using the Klenow fragment and then digested with XbaI. The vector fragment and the PCR amplicon were ligated and transformed into *E.coli* by electrotransformation. The resulting vector is designated pAMGY.

### Construction Of pLaC103

The *A. niger* AMGII cDNA clone (Boel et al., (1984), supra) is used as source for the construction of pLaC103 aimed at *S. cerevisiae* expression of the GII form of AMG.

The construction takes place in several steps, out lined below.

pT7-212 (EP37856/ US patent no. 5162498) is cleaved with XbaI, blunt-ended with Klenow DNA polymerase and dNTP. After cleavage with EcoRI the resulting vector fragment is purified from an agarose gel-electrophoresis and ligated with the 2.05 kb EcoR1-EcoRV fragment of pBoel53, thereby recreating the XbaI site in the EcoRV end of the AMG encoding fragment in the resulting plasmid pG2x.

In order to remove DNA upstream of the AMG cds, and furnish the AMG encoding DNA with an appropriate restriction endonuclease recognition site, the following construct was made:
The 930 bp EcoRI-PstI fragment of p53 was isolated and subjected to AluI cleavage, the resulting 771 bp Alu-PstI fragment was ligated into pBR322 with blunt-ended EcoRI site (see above) and cleaved with PstI In the resulting plasmid pBR-AMG', the EcoRI site was recreated just 34 bp from the initiation codon of the AMG cds.

From pBR-AMG' the 775 bp EcoRI - PstI fragment was isolated and joined with the 1151 bp PstI - XbaI fragment from pG2x in a ligation reaction including the XbaI - EcoRI vector fragment of pT7-212.

The resulting plasmid pT7GII was submitted to a BamHI cleavage in presence of alkaline phosphatase followed by partial SphI cleavage after inactivation of the phosphatase. From this reaction was the 2489 bp SphI-BamHI fragment, encompassing the S.c. TPI promoter linked to the AMGII cds.
The above fragment together with the 1052 bp BamHI fragment of pT7GII was ligated with the alkaline phosphatase treated vector fragment of pMT743 (EP37856/US 5162498), resulting from SphI-BamHI digestion. The resulting plasmid is pLaC103.

### Screening For Thermostable AMG Variants

The libraries are screened in the thermostable filter assay described below.

### Filter Assay For Thermostability

Yeast libraries are plated on a sandwich of cellulose acetate (OE 67, Schleicher & Schuell, Dassel, Germany) - and nitrocellulose filters (Protran-Ba 85, Schleicher & Schuell, Dassel, Germany) on SCFura⁻agar plates with 100 µg/ml ampicillin at 30°C for at least 72 hrs. The colonies are replica plated to PVDF filters (Immobilon-P, Millipore, Bedford) activated with methanol for 1 min or alternatively a Protran filter (no activation) and subsequently washed in 0.1 M NaAc and then incubated at room temperature for 2 hours. Colonies are washed from PVDF/Protran filters with tap water. Each filter sandwiches and PVDF/Protran filters are specifically marked with a needle before incubation in order to be able to localise positive variants on the filters after the screening. The PVDF filters with bound variants are transferred to a container with 0.1 M NaAc, pH 4.5 and incubated at 47°C or alternatively 67-69°C in case of Protran filters for 15 minutes. The sandwich of cellulose acetate and nitrocellulose filters on SC ura-agar plates are stored at room temperature until use. After incubation, the residual activities are detected on plates containing 5% maltose, 1% agarose, 50 mM NaAc, pH 4.5. The assay plates with PVDF filters are marked the same way as the filter sandwiches and incubated for 2 hrs. at 50°C. After removal of the PVDF filters, the assay plates are stained with Glucose GOD perid (Boehringer Mannheim GmbH, Germany). Variants with residual activity are detected on assay plates as dark green spots on white background. The improved variants are located on the storage plates. Improved variants are rescreened twice under the same conditions as the first screen.

### General Method For Random Mutagenesis By Use Of The DOPE Program

The random mutagenesis may be carried out by the following steps:
1. Select regions of interest for modification in the parent enzyme,
2. Decide on mutation sites and non-mutated sites in the selected region,
3. Decide on which kind of mutations should be carried out, e.g., with respect to the desired stability and/or performance of the variant to be constructed,
4. Select structurally reasonable mutations,
5. Adjust the residues selected by step 3 with regard to step 4.
6. Analyze by use of a suitable dope algorithm the nucleotide distribution.
7. If necessary, adjust the wanted residues to genetic code realism, e.*g.* taking into account constraints resulting from the genetic code, e.g. in order to avoid introduction of stop codons; the skilled person will be aware that some codon combinations cannot be used in practice and will need to be adapted
8. Make primers
9. Perform random mutagenesis by use of the primers
10. Select resulting glucoamylase variants by screening for the desired improved properties.

### Dope Algorithm

Suitable dope algorithms for use in step 6 are well known in the art. One such algorithm is described by Tomandl, D. et al., 1997, Journal of Computer-Aided Molecular Design 11:29-38. Another algorithm is DOPE (Jensen, LJ, Andersen, KV, Svendsen, A, and Kretzschmar, T (1998) Nucleic Acids Research 26:697-702).

### Method Of Extracting Important Regions For Temperature Activity Using Molecular Simulation.

The X-ray structure and/or the model-build structure of the enzyme of interest, here AMG, are subjected to molecular dynamics simulations. The molecular dynamics simulation are made using the CHARMM (from Molecular simulations (MSI)) program or other suitable programs, e.g., DISCOVER (from MSI). The dynamics are made in vacuum, or including crystal waters, or with the enzyme in question embedded in a suitable waters, e.g., a sphere or a box. The simulation are run for 300 picoseconds (ps) or more, e.g., 300-1200 ps. The isotropic fluctuations are extracted for the CA carbons of the structures and comparison between the structures are made. More details on how to get the isotropic fluctuations can be found in the CHARMM manual (available from MSI).

The molecular dynamics simulation can be carried out using standard charges on the chargeable amino acids. For instance, Asp and Glu is negatively charged and Lys and Arg are positively charged. This condition resembles the medium pH of approximately 7.0. To analyze a lower pH, titration of the molecule can be done to obtain the altered pKa's of the normal titrateable residues within pH 2-10; Lys, Arg, Asp, Glu, Tyr and His. Also Ser, Thr and Cys are titrateable but are not taking into account here. Here the altered charges due to the pH has been described as all Arg, Lys negative at high pH, and all Asp, Glu are uncharged. This imitates a pH around 4 to 5 where the titration Asp and Glu normally takes place.

Model building of the enzyme of interest can be obtained by using the HOMOLOGY model in the MSI program package. The crystal structure of *Aspergillus awamori* variant x100 can be found in, e.g., 3GLY and 1DOG in the Brookhaven database.

### EXAMPLES

### EXAMPLE 1

### Construction of AMG G2 variants

### Site-directed mutagenesis:

For the construction of variants of AMG G2 (SEQ ID NO: 2) the commercial kit, Chameleon double-stranded, site-directed mutagenesis kit was used according to the manufacturer's instructions.

The gene encoding the AMG G2 enzyme in question is located on pMT838 prepared by deleting the DNA between G2 nt. 1362 and G2 nt. 1530 in plasmid pCAMG91 (see Figure 1) comprising the AMG G1 form.

In accordance with the manufacturer's instructions the ScaI site of the Ampicillin gene of pMT838 was changed to a Mlul site by use of the following primer:
7258: 5'p gaa tga ctt ggt tga cgc gtc acc agt cac 3' (SEQ ID NO: 3).
(Thus changing the ScaI site found in the ampicillin resistance gene and used for cutting to a MluI site). The pMT838 vector comprising the AMG gene in question was then used as a template for DNA polymerase and oligo 7258 (SEQ ID NO: 3) and 21401 (SEQ ID NO: 4).

Primer no. 21401 (SEQ ID NO: 4) was used as the selection primer.
21401: 5'p gg gga tca tga tag gac tag cca tat taa tga agg gca tat acc acg cct tgg acc tgc gtt ata gcc 3'
(Changes the ScaI site found in the AMG gene without changing the amino acid sequence).
The desired mutation (e.g., the introduction of a cystein residue) is introduced into the AMG gene in question by addition of appropriate oligos comprising the desired mutation.

The primer 107581 was used to introduce T12P
107581: 5' pgc aac gaa gcg ccc gtg gct cgt ac 3' (SEQ ID NO: 5)

The mutations are verified by sequencing the whole gene. The plasmid was transformed into *A. oryzae* using the method described above in the "Materials & Methods" section. The variant was fermented and purified as described above in the "Materials & Methods" section.

### EXAMPLE 2

### Construction, by localized random, doped mutagenesis, of A. niger AMG variants having improved thermostability compared to the parent enzyme

To improve the thermostability of the A. *niger* AMG random mutagenesis in pre-selected region was performed.

Residue:
Region: L19-G35
Region: A353-V374

The DOPE software (see Materials and Methods) was used to determine spiked codons for each suggested change in the above regions minimizing the amount of stop codons (see table 1). The exact distribution of nucleotides was calculated in the three positions of the codon to give the suggested population of amino acid changes. The doped regions were doped specifically in the indicated positions to have a high chance of getting the desired residues, but still allow other possibilities.

The first column is the amino acid to be mutated, the second column is the percentage of wild type and the third column defined the new amino acid(s).

**Table 1**

| Doping in L19-G35 | | |
|---|---|---|
| L19 | 90% | N |
| N20 | 95% | T |
| N21 | Constant | |
| I22 | Constant | |
| G23 | 95% | A |
| A24 | 90% | S,T |
| D25 | 93% | S,T,R |
| G26 | 95% | A |
| A27 | 90% | S,T |
| W28 | <80% | R,Y |
| V29 | Constant | |
| S30 | 93% | T,N |
| G31 | 95% | A |
| A32 | 95% | V |
| D33 | 80% | R,K,H |
| S34 | 90% | N |
| G35 | Constant | |

The resulting doped oligonucleotide strand is shown in table 2 as sense strand: with the primer sequence, the wild type nucleotide sequence, the parent amino acid sequence and the distribution of nucleotides for each doped position.

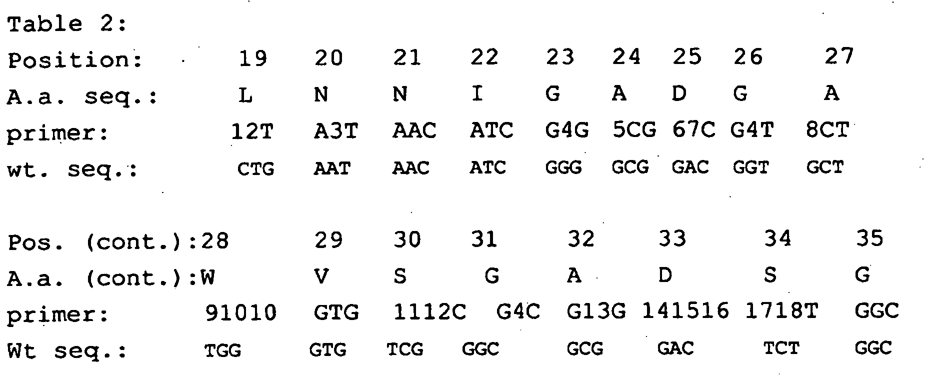

Distribution of nucleotides for each doped position.
1: A10,C90
2 : A6, T94
3 : A95,C5
4 : G95, C5
5 : G91,A3,T3,C3
6 : G95, A3, C2
7 : G3, A95, C2
8 : G92, A4, T4
9:A3,T97
10:G95,T5
11:G3, A97
12:G95,A2,C3
13:T5,C95
14 : G88, A8, C4
15:G7,A93
16:G4,C96
17:G4,A96
18:G95,A2,C3

### Forward primer (SEQ ID NO: 6):

FAMGII '5-C GAA GCG ACC GTG GCT CGT ACT GCC ATC 12T A3T AAC ATC G4G 5CG 67C G4T 8CT 91010 GTG 1112C G4C G13G 141516 1718T GGC ATT GTC GTT GCT AGT CCC AGC ACG GAT AAC-3'

### Reverse primer (SEQ ID NO: 7):

RAMG1: 5'-GAT GGC AGT ACG AGC CAC GGT CGC TTC G-3'

**Table 3_**

| Doping in region A353-V374 : | | |
|---|---|---|
| A353 | <80% | D, E, Q, N, Y |
| L354 | 90% | Q, E |
| Y355 | 90% | N,Q |
| S356 | 90% | T,D,N |
| G357 | 80% | P,A,S,T |
| A358 | 93% | S |
| A359 | 90% | S,T,N |
| T360 | 90% | R,K |
| G361 | 85% | A,S,T |
| T362 | 90% | S |
| Y363 | Constant | |
| S364 | 93% | D |
| S365 | 93% | N,Q,K |
| S366 | 93% | P,D |
| S367 | Constant | |
| S368 | 93% | D,N,T |
| T369 | 93% | Q, E |
| Y370 | Constant | |
| S371 | 93% | N |
| S372 | 93% | N,T |
| I373 | Constant | |
| V374 | 93% | N,Y,H |

The resulting doped oligonucleotide strand is shown in table 4 as sense strand: with the primer sequence, wild type nucleotide sequence, the parent amino acid sequence and the distribution of nucleotides for each doped position.

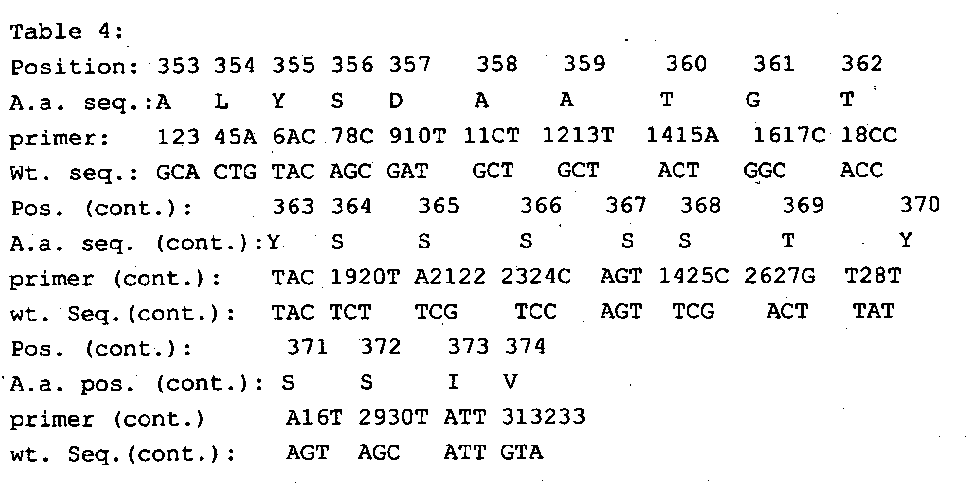

Distribution of nucleotides for each doped position.
1 : G91,A3,T3,C3
2 : A13,C87
3:A40,T60
4 : G3,A3,C94
5 : A6,T94
6 : G4, A4, T92
7 : G2,A96,C2
8 : G93, A3.5, C3.5
9 : G87, A8, C5
10 : A84, C16
11:G93,T7
12:G92,A5,T3
13:A3,C97
14:G3,A97
15:G2,A2,T4,C92
16:G93,A7
17:G93,C7
18:A90,T10
19:G4,A96
20:G95,A5
21 : G96,A4
22:G3,C97
23:G2,A1,T95,C2
24:A3,C97
25:G95,A3,C2
26:G2,A96,C2
27:A5,C95
28:A95,T5
29:G2,A98
30:G94,A4,C2
31: G94, A3, T1, C2
32:A4,T96
33:A20,C80

### Primer: FAMGIV (SEQ ID NO: 8)

### Primer RAMGVI (SEQ ID NO: 9)

5'-ctt gaa gaa gtc cag cga cac-3'

### Random mutagenesis

The spiked oligonucleotides apparent from Table 2 and 3 (which by a common term is designated FAMG) and reverse primers RAMG for the L19-G35 region and specific SEQ ID NO: 2 primers covering the N-terminal (FG2: 5'- CAT CCC CAG GAT CCT TAC TCA GCA ATG-3' (SEQ ID NO: 10) and C-terminal (RG2: 5'- CTC AAA CGA CTC ACC AGC CTC TAG AGT (SEQ ID NO: 11) are used to generate PCR-library-fragments by the overlap extension method (Horton et al., Gene, 77 (1989), pp. 61-68) with an overlap of 21 base pairs. Plasmid pAMGY is template for the Polymerase Chain Reaction. The PCR fragments are cloned by homologous recombination in the E. *coli*/yeast shuttle vector pAMGY (see Materials and Methods).

### Screening

The library was screened in the thermostability filter assays using a Protran filter and incubating at 67-69°C as described in the "Material & Methods" section above

### EXAMPLE 3

### Construction, by PCR shuffling spiked with DNA oligos, of A. niger AMG variants having improved thermostability compared to the parent enzyme.

The polymerase chain reaction (PCR) method was used to prepare DNA fragments carrying the AMG gene and flanking regions. Approximately 10 ug DNA was digested with Dnase, and run on a 2 % agarose gel. Fragments of 50-150 bp were purified from the gel. Approximately 1 ug purified fragments were mixed with a 5-15 fold molar excess of oligos carrying the desired mutations. The oligos were of the following kind (for the construction of Hklib1, Hklib2, Hklib3 etc., respectively):

### Hklib1:

### Hk1-T2X:

5'-ATGTGATTTCCAAGCGCGCG**VNN**TTGGATTCATGGTTGAGCAA

### Hk1-N9X:

5'-CCTTGGATTCATGGTTGAGC**VNN**GAAGCGACCGTGGCTCGTAC

### Hk1-A11X:

5'-ATTCATGGTTGAGCAACGAA**VNN**ACCGTGGCTCGTACTGCCAT

### Hk1-L66X:

5'-TCCTCAAGACCCTCGTCGAT**VNN**TTCCGAAATGGAGATACCAG

### Hk1-S386X:

5'-CTTTCGCCGATGGCTTCGTC**VNN**ATTGTGGAAACTCACGCCGC

### Hk1-E389X:

5'-ATGGCTTCGTCTCTATTGTG**VNN**ACTCACGCCGCAAGCAACGG

### Hk1-T390X:

5'-GCTTCGTCTCTATTGTGGAA**VNN**CACGCCGCAAGCAACGGCTC

### Hkl-A393X:

5'-CTATTGTGGAAACTCACGCC**VNN**AGCAACGGCTCCATGTCCGA

### Hkl-S394X:

5'-TTGTGGAAACTCACGCCGCA**VNN**AACGGCTCCATGTCCGAGCA

### Hk1-N395X:

5'-TGGAAACTCACGCCGCAAGC**VNN**GGCTCCATGTCCGAGCAATA

### Hk1-G396X:

5'-AAACTCACGCCGCAAGCAAC**VNN**TCCATGTCCGAGCAATACGA

### Hk1-K404X:

5'-CCATGTCCGAGCAATACGAC**VNN**TCTGATGGCGAGCAGCTTTC

### Hk1-D406X:

5'-CCGAGCAATACGACAAGTCT**VNN**GGCGAGCAGCTTTCCGCTCG

### Hk1-E408X:

5'-AATACGACAAGTCTGATGGC**VNN**CAGCTTTCCGCTCGCGACCT

### Hk1-L410X:

5'-ACAAGTCTGATGGCGAGCAG**VNN**TCCGCTCGCGACCTGACCT

### Hk1-L423X:

5'-CCTGGTCTTATGCTGCTCTG**VNN**ACCGCCAACAACCGTCGTAA

### Hk1-N426X:

5'-ATGCTGCTCTGCTGACCGCC**VNN**AACCGTCGTAACTCCGTCGTG

### Hk1-N427X:

5'-CTGCTCTGCTGACCGCCAAC**VNN**CGTCGTAACTCCGTCGTGCCT

### Hk1-Y402X:

5'-ACGGCTCCATGTCCGAGCAA**NNC**GACAAGTCTGATGGCGAGCAGCT

### Hklib2:

### Hk2-L234X-SENSE:

5'-CTGGACCGGCAGCTTCATT**NN**KGCCAACTTCGATAGCAGCC

### Hk2-A235S-ANTISENSE:

5 '-GAACGGCTGCTATCGAAGTT**AGA**CAGAATGAAGCTGCCGGTC

### Hk2-F237X-SENSE:

5-CAGCTTCATTCTGGCCAAC**NAT**GATAGCAGCCGTTCCGGCA

### Hk2-D238T-ANTISENSE:

5'-CCTTGCCGGAACGGCTGCT**AGT**GAAGTTGGCCAGAATGAAGC

### Hk2-D238S-ANTISENSE:

5'-CCTTGCCGGAACGGCTGCTAG**AGA**AGTTGGCCAGAATGAAGC

### Hk2-S239X-SENSE:

5'-TCATTCTGGCCAACTTCGAT**NNC**AGCCGTTCCGGCAAGGACG

### Hk2-S240G-ANTISENSE:

5'-TTGCGTCCTTGCCGGAACG**ACC**GCTATCGAAGTTGGCCAGAA

### Hk2-S242X-ANTISENSE:

5'-GGGTGTTTGCGTCCTTGCC**AKN**ACGGCTGCTATCGAAGTTG

### Hk2-G243X-ANTISENSE:

5'-GGAGGGTGTTTGCGTCCTT**AKN**GGAACGGCTGCTATCGAA

### Hk2-K244R-SENSE:

5'-CGATAGCAGCCGTTCCGGC**AGA**GACGCAAACACCCTCCTGG

### Hk2-T310V-ANTISENSE:

5'-ACGGGTTGCCGTTGTAGTA**AAC**GTCCTCAGGGTACCGACCC

### Hk2-T310S-ANTISENSE:

5'-ACGGGTTGCCGTTGTAGTA**AGA**GTCCTCAGGGTACCGACCC

### Hk2-Y311N-SENSE:

5'-TCGGTACCCTGAGGACACG**AAT**TACAACGGCAACCCGTGGT

### Hk2-Y312Q-ANTISENSE:

5'-GGAACCACGGGTTGCCGTT**TTG**GTACGTGTCCTCAGGGTAC

### Hk2-Y312N-ANTISENSE:

5'-GGAACCACGGGTTGCCGTT**ATT**GTACGTGTCCTCAGGGTAC

### Hk2-N313T-SENSE:

5'-CCCTGAGGACACGTACTAC**ACT**GGCAACCCGTGGTTCCTGT

### Hk2-N313S-SENSE:

5'-CCCTGAGGACACGTACTAC**TCT**GGCAACCCGTGGTTCCTGT

### Hk2-N313G-SENSE:

5'-CCCTGAGGACACGTACTAC**GGT**GGCAACCCGTGGTTCCTGT

### Hk2-N315Q-ANTISENSE:

5'-AGGTGCACAGGAACCACGG**TTG**GCCGTTGTAGTACGTGTCC

### Hk2-N315E-ANTISENSE:

5'-AGGTGCACAGGAACCACGG**TTC**GCCGTTGTAGTACGTGTCC

### Hk2-N315R-ANTISENSE:

5'-AGGTGCACAGGAACCACGG**TCT**GCCGTTGTAGTACGTGTCC

### Hk2-F318Y-ANTISENSE:

5'-CGGCAGCCAAGGTGCACAG**ATA**CCACGGGTTGCCGTTGTAG

### Hk2-Q409P-SENSE:

5'-CGACAAGTCTGATGGCGAG**CCA**CTTTCCGCTCGCGACCTGA

### Hklib3:

### Hk3-D336X-SENSE:

5'-CGATGCTCTATACCAGTGG**NNK**AAGCAGGGGTCGTTGGAGG

### Hk3-K337X-SENSE:

5'-TGCTCTATACCAGTGGGAC**NNK**CAGGGGTCGTTGGAGGTCA

### Hk3-Q338X-ANTISENSE :

5'-CTGTGACCTCCAACGACCC**GNN**CTTGTCCCACTGGTATAGA

### Hk3-G339X-SENSE:

5'-ATACCAGTGGGACAAGCAG**NCU**TCGTTGGAGGTCACAGATG

### Hk3-S340X'-ANTISENSE:

5'-ACACATCTGTGACCTCCAA**ANT**CCCCTGCTTGTCCCACTGG

### Hk3-S340X"-ANTISENSE:

5'-ACACATCTGTGACCTCCAA**ANC**CCCCTGCTTGTCCCACTGG

### Hk3-L341X-SENSE:

5'-GTGGGACAAGCAGGGGTCG**NUU**GAGGTCACAGATGTGTCGC

### Hk3-K352Q-SENSE:

5'-TGTGTCGCTGGACTTCTTC**CAA**GCACTGTACAGCGATGCTG

### Hk3-K352R-SENSE:

5'-TGTGTCGCTGGACTTCTTC**AGA**GCACTGTACAGCGATGCTG

### Hk3-A353D-ANTISENSE:

5'-TAGCAGCATCGCTGTAC**AGA**TCCTTGAAGAAGTCCAGCGAC

### Hk3-A353S-ANTISENSE:

5'-TAGCAGCATCGCTGTACAG**AGA**CTTGAAGAAGTCCAGCGAC

### Hk3-S356P-SENSE:

5'-ACTTCTTCAAGGCACTGTAC**CCA**GATGCTGCTACTGGCACCT

### Hk3-S356N-SENSE:

5'-ACTTCTTCAAGGCACTGTAC**AAU**GATGCTGCTACTGGCACCTA

### Hk3-S356D-SENSE:

5'-ACTTCTTCAAGGCACTGTAC**GAU**GATGCTGCTACTGGCACCTA

### Hk3-D357S-ANTISENSE:

5'-GAGTAGGTGCCAGTAGCAGC**AGA**GCTGTACAGTGCCTTGAAGA

### Hk3-A359S-SENSE:

5'-GGCACTGTACAGCGATGCT**TCT**ACTGGCACCTACTCTTCGT

### Hk3-T360V-ANTISENSE:

5'-TGGACGAAGAGTAGGTGCC**AAC**AGCAGCATCGCTGTACAGT

### Hk3-G361X-SENSE:

5'-TGTACAGCGATGCTGCTACT**NCT**ACCTACTCTTCGTCCAGTTC

### Hk3-T362R-ANTISENSE:

5'-GTCGAACTGGACGAAGAGTA**TCT**GCCAGTAGCAGCATCGCTG

### Hk3-S364X-SENSE:

5'-TGCTGCTACTGGCACCTAC**NNK**TCGTCCAGTTCGACTTATAG

### Hk3-S65X-SENSE:

5'-TGCTACTGGCACCTACTCT**NNK**TCCAGTTCGACTTATAGTAG

### Hk3-S366T-ANTISENSE:

5'-ATGCTACTATAAGTCGAACT**AGT**CGAAGAGTAGGTGCCAGTA

### Hk3-S368X-ANTISENSE:

5'-TCTACAATGCTACTATAAGT**AGN**ACTGGACGAAGAGTAGGTG

### Hk3-T369X-SENSE:

5 '-CTACTCTTCGTCCAGTTCG**NNK**TATAGTAGCATTGTAGATGCC

### Hk3-S371X-ANTISENSE:

5'-TTCACGGCATCTACAATGCT**ATN**ATAAGTCGAACTGGACGAAG

### Hk3-S372X-SENSE:

5'-CGTCCAGTTCGACTTATAGT**NNTA**TTGTAGATGCCGTGAAGAC

To the mix of Dnase treated DNA and oligos was added nucleotides, PCR buffer and Taq/Pwo polymerase. A PCR assembly reaction was performed, using first 94 oC for 2 min., then 35-40 cycles with the following incubation times: 94°C, 30 sec.; 45 °C, 30 sec.; 72°C, 60 sec ; then finally 72°C for 5 min. An PCR amplification reaction was performed with 1 uL of the assembly reaction as template, and adding primers that anneal to the regions flanking the AMG gene. Parameters: first 94 °C for 2 min., then 35-40 cycles with the following incubation times: 94°C, 30 sec.; 55°C, 30 sec.; 72 °C, 90 sec ; then finally 72°C for 10 min.

The resulting PCR product was purified from a 1% agarose gel, mixed with linearized vector and transformed into competent yeast cells, as described above.

### EXAMPLE 4 (Referential)

### Specific Activity

AMG G2 variants were constructed as described above in Example 1. The specific activity as k_{cat} were measured on purified samples at pH 4.3, 37°C, using maltose and maltohepatose as substrate as described in the "Materials & Methods" section above. The specific activity as AGU/mg were also measured at pH 4.3, 37°C, using maltose as substrate as described in the "Materials & Methods" section above.

| | Kcat (sec.-1) | |
|---|---|---|
| Variant | Maltose | Maltoheptaose |
| AMG G2 (wt) | 6.0 | 38 |
| N110T | 9.7 | 27.8 |
| V111P | 12.0 | 43.2 |
| S119P | 6.2 | 44.0 |
| G127A | 21.0 | 40.0 |
| G207N | 30.5 | 36.3 |

| Variant | AGU/mg |
|---|---|
| AMG G2 (wild type) | 1.8 |
| N110T | 3.5 |
| V111P | 3.1 |
| S119P | 2.1 |
| G127A | 5.8 |
| G207N | 5.7 |
| L3N | 2.3 |
| S56A | 2.6 |
| A102* | 2.5 |
| D403S | 2.2 |
| I18V+T51S+S56A+V59T+L60A | 3.3 |
| S119P+Y402F | 2.7 |
| S119P+I189T+Y223F+F227Y+Y402F | 3.0 |

### EXAMPLE 5 (Referential)

### Thermostability at 70°C

An AMG G2 S119P variant was constructed using the approach described in Example 1.

The thermostability was determined as T_{1/2} using Method I, and as % residual activity after incubation for 30 minutes in 50 mM NaOAc, pH 4.5, 70°C, 0.2 AGU/ml, as described in the "Material & Methods" section above. The result of the tests are listed in the Table below and compared to the wild-type A. *niger* AMG G2.

| *A. niger* AMG (Enzyme) | Residual activity (%) | T_{1/2} (min.) |
|---|---|---|
| S119P variant | 22 | 17 |
| wild-type (SEQ ID NO: 2) | 13 | 8 |

### EXAMPLE 6

### Thermostability at 68°C

AMG G2 variants were constructed using the approach described in Example 3, except for variants nos. 1 and 2 in the Table below, which were prepared by shuffling as described in WO 95/22625 (from Affymax Technologies N.V.). The thermostability was determined as T½ using method I at 68°C as described in the "Materials & Methods" section and compared to the wild-type A. *niger* AMG G2 under the same conditions. Evaluation of variants were performed on culture broth after filtration of the supernatants.

| | Variant | T½ (min) | T½ *A. niger* AMG G2 (wild type) (min) |
|---|---|---|---|
| 1* | A246T+T72I | 11.3 | 8.5 |
| 2* | G447S+S119P | 11.4 | 7.9 |
| 3* | E408R+A425T+S465P+T494A | 8.6 | 8.1 |
| 4 | E408R+S386N | 12.6 | 8.9 |
| 5* | T2P | 9.3 | 8.5 |
| 6 | T2Q+A11P+S394R | 10.7 | 8.5 |
| 7* | T2H | 9.5 | 8.9 |
| 8 | A11E+E408R | 12.7 | 9.3 |
| 9 | T2M+N9A+T390R+D406N+L410R | 10.7 | 8.5 |
| 10 | A393R | 17.7 | 8.4 |
| 11 | T2R+S386R+A393R | 14.1 | 8.4 |
| 12 | A393R+L410R | 14.7 | 7.9 |
| 13 | A1V+L66R+Y402F+N427S+S486G | 11.7 | 8.5 |
| 14* | T2K+S30P+N427M+S444G+V470M | 11.4 | 8.4 |

| | | | |
|---|---|---|---|
| * not a variant of the invention | | | |

| Thermostability at 70°C on purified samples. | | |
|---|---|---|
| | Enzyme | T½ (min) |
| 15 | AMG G2 (wild type) | 7.4 |
| 16 | T2E+T379A+S386K+A393R | 11.6 |
| 17 | E408R+S386N | 10.2 |
| 18 | T2Q+A11P+S394R | 9.8 |
| 19 | A1V+L66R+Y402F+N427S+S486G | 14.1 |
| 20 | A393R | 14.6 |
| 21 | T2R+S386R+A393R | 14.1 |
| 22 | A393R+L410R | 12.9 |
| 23 | Y402F | 10.1 |

### EXAMPLE 7

### Thermostability at 68°C

AMG G2 variants were constructed by shuffling using the approach described in Example 3 followed by shuffling of positive variants.

The thermostability was determined as T½ using method I at 68°C as described in the "Materials & Methods" section and compared to the wild-type A. *niger* AMG G2 under the same conditions. Evaluation of variants were performed on culture broth after filtration of the supernatants.

| | Variant | T½ (min) | T½ A. niger AMG G2 (wild type) (min) |
|---|---|---|---|
| 24 | PLASDⁱ+V59A+A393R+T490A | 27.2 | 6.8 |

i = N-terminal extension

### EXAMPLE 8 (Referential)

### Thermostability at 68°C

AMG G2 variants were constructed using the approach described in Example 3. The thermostability was determined as T1/2 using method I at 68°C as described in the "Materials & Methods" section and compared to the wild-type A. *niger* AMG G2 under the same conditions. Evaluation of variants were performed on culture broth after filtration of the supernatants.

| | Variant | T½ (min) | T½ *A. niger* AMG G2 wild-type (min) |
|---|---|---|---|
| 25 | D357S+T360V+S371H | 6.6 | 5.9 |
| 26 | N313G+F318Y | 8.9 | 5.9 |
| 27 | S356P+S366T | 7.3 | 5.8 |
| 28 | S340G+D357S+T360V+S386P | 7.2 | 5.8 |

### EXAMPLE 9

### Thermostability at 70°C

An AMG G2 variants was constructed using the approach described in Example 1 and evaluated as semi-purified (filtration of culture broth followed by desalting on a G-25 column) samples.

The thermostability was determined as % residual activity using Method I in 50 mM NaOAc, pH 4.5, 70°C, as described in the "Material & Methods" section above. The result of the test is listed in the Table below and compared to the wild-type A. *niger* AMG G2.

| | Enzyme | T½ (min) |
|---|---|---|
| 29 | AMG G2 (wild type) | 7 |
| 30 | Y402F+S411V | 60 |
| 31 | S119P+Y402F+S411V | 115 |
| 32 | S119P+Y312Q+Y402F+T416H | 50 |

### EXAMPLE 10

### Thermostability at 70°C in presence of 30% glucose

AMG G2 variants were constructed using the approach described in Example 3.

The thermostability was determined as T½ using method II at 70°C as described in the "Materials & Methods" section and compared to the wild-type A. *niger* AMG G2 under the same conditions.

| | Enzyme | T½ (hr) |
|---|---|---|
| 33 | AMG G2 (wild type) | 1.5 |
| 34 | Y402F | 2.5 |
| 35 | A393R | 4.0 |
| 36 | T2R+S386R+A393R | 2.0 |
| 37 | PLASD(N-terminal)+V59A+A393R+T490A | 16.0 |

### EXAMPLE 11

### Saccharification performance of AMG variants S119P+Y402F+S411V and PLASD(N-terminal)+V59A+A393R+T490A, respectively.

Saccharification performance of the AMG variants S119P+Y402F+S411V and PLASD(N-terminal)+V59A+A393R+T490A, respectively, both having improved thermostability are tested at 70°C as described below.

Reference enzyme is the wild-type A. *niger* AMG G2. Saccharification is run under the following conditions:

| | |
|---|---|
| Substrate | 10 DE Maltodextrin, approx. 30% DS (w/w) |
| Temperature | 70°C |
| Initial pH | 4.3 (at 70°C) |
| Enzyme dosage | 0.24 AGU/g DS |

### Saccharification

The substrate for saccharification is made by dissolving maltodextrin (prepared from common corn) in boiling Milli-Q water and adjusting the dry substance to approximately 30% (w/w). pH is adjusted to 4.3. Aliquots of substrate corresponding to 15 g dry solids are transferred to 50 ml blue cap glass flasks and placed in a water bath with stirring. Enzymes are added and pH re-adjusted if necessary. The experiment is run in duplicate. Samples are taken periodically and analysed at HPLC for determination of the carbohydrate composition.

### SEQUENCE LISTING

(1) GENERAL INFORMATION:
   (i) APPLICANT:
      (A) NAME: NOVO NORDISK A/S
      (B) STREET: Novo Allé
      (C) CITY: DK-2880 Bagsvaerd
      (E) COUNTRY: Denmark
      (F) POSTAL CODE (ZIP): DK-2880
      (G) TELEPHONE: +45 4444 8888
      (H) TELEFAX: +45 4449 3256
   (ii) TITLE OF INVENTION: Glucoamylase variants
   (iii) NUMBER OF SEQUENCES: 81
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.25 (EPO)
(2) INFORMATION FOR SEQ ID NO: 1:
   (i) SEQUENCE CHARACTERISTICS:
      (A) LENGTH: 1605 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "cDNA"
   (vi) ORIGINAL SOURCE:
      (B) STRAIN: Aspergillus niger
   (ix) FEATURE:
      (A) NAME/KEY: sig_peptide
      (B) LOCATION : 1..72
   (ix) FEATURE: _
      (A) NAME/KEY: mat_peptide
      (B) LOCATION:73..1602
   (ix) FEATURE:
      (A) NAME/KEY: CDS
      (B) LOCATION:1..1602
   (xi) SEQUENCE DESCRIPTION: SEQ ID NO: 1:
   ATG TCG TTC CGA TCT CTA CTC GCC CTG AGC GGC CTC GTC TGC ACA GGG      48
   Met Ser Phe Arg Ser Leu Leu Ala Leu Ser Gly Leu Val Cys Thr Gly
   -24             -20                 -15                 -10
   TTG GCA AAT GTG ATT TCC AAG CGC GCG ACC TTG GAT TCA TGG TTG AGC      96
   Leu Ala Asn Val Ile Ser Lys Arg Ala Thr Leu Asp Ser Trp Leu Ser
                -5                   1               5
   AAC GAA GCG ACC GTG GCT CGT ACT GCC ATC CTG AAT AAC ATC GGG GCG     144
   Asn Glu Ala Thr Val Ala Arg Thr Ala Ile Leu Asn Asn Ile Gly Ala
        10                  15                  20
   GAC GGT GCT TGG GTG TCG GGC GCG GAC TCT GGC ATT GTC GTT GCT AGT     192
   Asp Gly Ala Trp Val Ser Gly Ala Asp Ser Gly Ile Val Val Ala Ser
    25                  30                  35                  40
   CCC AGC ACG GAT AAC CCG GAC TAC TTC TAC ACC TGG ACT CGC GAC TCT     240
   Pro Ser Thr Asp Asn Pro Asp Tyr Phe Tyr Thr Trp Thr Arg Asp Ser
                    45                  50                  55
   GGT CTC GTC CTC AAG ACC CTC GTC GAT CTC TTC CGA AAT GGA GAT ACC     288
   Gly Leu Val Leu Lys Thr Leu Val Asp Leu Phe Arg Asn Gly Asp Thr
                60                  65                  70
   AGT CTC CTC TCC ACC ATT GAG AAC TAC ATC TCC GCC CAG GCA ATT GTC     336
   Ser Leu Leu Ser Thr Ile Glu Asn Tyr Ile Ser Ala Gln Ala Ile Val
            75                  80                  85
   CAG GGT ATC AGT AAC CCC TCT GGT GAT CTG TCC AGC GGC GCT GGT CTC     384
   Gln Gly Ile Ser Asn Pro Ser Gly Asp Leu Ser Ser Gly Ala Gly Leu
        90                  95                 100
   GGT GAA CCC AAG TTC AAT GTC GAT GAG ACT GCC TAC ACT GGT TCT TGG     432
   Gly Glu Pro Lys Phe Asn Val Asp Glu Thr Ala Tyr Thr Gly Ser Trp
   105                 110                 115                 120
   GGA CGG CCG CAG CGA GAT GGT CCG GCT CTG AGA GCA ACT GCT ATG ATC        480
   Gly Arg Pro Gln Arg Asp Gly Pro Ala Leu Arg Ala Thr Ala Met Ile
                   125                 130                 135
   GGC TTC GGG CAG TGG CTG CTT GAC AAT GGC TAC ACC AGC ACC GCA ACG        528
   Gly Phe Gly Gln Trp Leu Leu Asp Asn Gly Tyr Thr Ser Thr Ala Thr
               140                 145                 150
   GAC ATT GTT TGG CCC CTC GTT AGG AAC GAC CTG TCG TAT GTG GCT CAA        576
   Asp Ile Val Trp Pro Leu Val Arg Asn Asp Leu Ser Tyr Val Ala Gln
           155                 160                 165
   TAC TGG AAC CAG ACA GGA TAT GAT CTC TGG GAA GAA GTC AAT GGC TCG        624
   Tyr Trp Asn Gln Thr Gly Tyr Asp Leu Trp Glu Glu Val Asn Gly Ser
       170                 175                 180
   TCT TTC TTT ACG ATT GCT GTG CAA CAC CGC GCC CTT GTC GAA GGT AGT        672
   Ser Phe Phe Thr Ile Ala Val Gln His Arg Ala Leu Val Glu Gly Ser
   185                 190                 195                 200
   GCC TTC GCG ACG GCC GTC GGC TCG TCC TGC TCC TGG TGT GAT TCT CAG        720
   Ala Phe Ala Thr Ala Val Gly Ser Ser Cys Ser Trp Cys Asp Ser Gln
                   205                 210                 215
   GCA CCC GAA ATT CTC TGC TAC CTG CAG TCC TTC TGG ACC GGC AGC TTC        768
   Ala Pro Glu Ile Leu Cys Tyr Leu Gln Ser Phe Trp Thr Gly Ser Phe
               220                 225                 230
   ATT CTG GCC AAC TTC GAT AGC AGC CGT TCC GGC AAG GAC GCA AAC ACC        816
   Ile Leu Ala Asn Phe Asp Ser Ser Arg Ser Gly Lys Asp Ala Asn Thr
           235                 240                 245
   CTC CTG GGA AGC ATC CAC ACC TTT GAT CCT GAG GCC GCA TGC GAC GAC        864
   Leu Leu Gly Ser Ile His Thr Phe Asp Pro Glu Ala Ala Cys Asp Asp
       250                 255                 260
   TCC ACC TTC CAG CCC TGC TCC CCG CGC GCG CTC GCC AAC CAC AAG GAG      912
   Ser Thr Phe Gln Pro Cys Ser Pro Arg Ala Leu Ala Asn His Lys Glu
   265                 270                 275                 280
   GTT GTA GAC TCT TTC CGC TCA ATC TAT ACC CTC AAC GAT GGT CTC AGT      960
   Val Val Asp Ser Phe Arg Ser Ile Tyr Thr Leu Asn Asp Gly Leu Ser
                   285                 290                 295
   GAC AGC GAG GCT GTT GCG GTG GGT CGG TAC CCT GAG GAC ACG TAC TAC     1008
   Asp Ser Glu Ala Val Ala Val Gly Arg Tyr Pro Glu Asp Thr Tyr Tyr
               300                 305                 310
   AAC GGC AAC CCG TGG TTC CTG TGC ACC TTG GCT GCC GCA GAG CAG TTG     1056
   Asn Gly Asn Pro Trp Phe Leu Cys Thr Leu Ala Ala Ala Glu Gln Leu
           315                 320                 325
   TAC GAT GCT CTA TAC CAG TGG GAC AAG CAG GGG TCG TTG GAG GTC ACA     1104
   Tyr Asp Ala Leu Tyr Gln Trp Asp Lys Gln Gly Ser Leu Glu Val Thr
       330                 335                 340
   GAT GTG TCG CTG GAC TTC TTC AAG GCA CTG TAC AGC GAT GCT GCT ACT     1152
   Asp Val Ser Leu Asp Phe Phe Lys Ala Leu Tyr Ser Asp Ala Ala Thr
   345                 350                 355                 360
   GGC ACC TAC TCT TCG TCC AGT TCG ACT TAT AGT AGC ATT GTA GAT GCC     1200
   Gly Thr Tyr Ser Ser Ser Ser Ser Thr Tyr Ser Ser Ile Val Asp Ala
                   365                 370                 375
   GTG AAG ACT TTC GCC GAT GGC TTC GTC TCT ATT GTG GAA ACT CAC GCC     1248
   Val Lys Thr Phe Ala Asp Gly Phe Val Ser Ile Val Glu Thr His Ala
               380                 385                 390
   GCA AGC AAC GGC TCC ATG TCC GAG CAA TAC GAC AAG TCT GAT GGC GAG     1296
   Ala Ser Asn Gly Ser Met Ser Glu Gln Tyr Asp Lys Ser Asp Gly Glu
           395                 400                 405
   CAG CTT TCC GCT CGC GAC CTG ACC TGG TCT TAT GCT GCT CTG CTG ACC     1344
   Gln Leu Ser Ala Arg Asp Leu Thr Trp Ser Tyr Ala Ala Leu Leu Thr
       410                 415                 420
   GCC AAC AAC CGT CGT AAC TCC GTC GTG CCT GCT TCT TGG GGC GAG ACC 1392
   Ala Asn Asn Arg Arg Asn Ser Val Val Pro Ala Ser Trp Gly Glu Thr
   425 430 435 440
   TCT GCC AGC AGC GTG CCC GGC ACC TGT GCG GCC ACA TCT GCC ATT GGT 1440
   Ser Ala Ser Ser Val Pro Gly Thr Cys Ala Ala Thr Ser Ala Ile Gly
   445 450 455
   ACC TAC AGC AGT GTG ACT GTC ACC TCG TGG CCG AGT ATC GTG GCT ACT 1488
   Thr Tyr Ser Ser Val Thr Val Thr Ser Trp Pro Ser Ile Val Ala Thr
   460 465 470
   GGC GGC ACC ACT ACG ACG GCT ACC CCC ACT GGA TCC GGC AGC GTG ACC 1536
   Gly Gly Thr Thr Thr Thr Ala Thr Pro Thr Gly Ser Gly Ser Val Thr
   475 480 485
   TCG ACC AGC AAG ACC ACC GCG ACT GCT AGC AAG ACC AGC ACC ACG ACC 1584
   Ser Thr Ser Lys Thr Thr Ala Thr Ala Ser Lys Thr Ser Thr Thr Thr
   490 495 500
   CGC TCT GGT ATG TCA CTG TGA 1605
   Arg Ser Gly Met Ser Leu
   505 510
(2) INFORMATION FOR SEQ ID NO: 2:
   (i) SEQUENCE CHARACTERISTICS:
      (A) LENGTH: 534 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Ser Phe Arg Ser Leu Leu Ala Leu Ser Gly Leu Val Cys Thr Gly
   -24 -20 -15 -10
   Leu Ala Asn Val Ile Ser Lys Arg Ala Thr Leu Asp Ser Trp Leu Ser
   -5 1 5
   Asn Glu Ala Thr Val Ala Arg Thr Ala Ile Leu Asn Asn Ile Gly Ala
   10 15 20
   Asp Gly Ala Trp Val Ser Gly Ala Asp Ser Gly Ile Val Val Ala Ser
   25 30 35 40
   Pro Ser Thr Asp Asn Pro Asp Tyr Phe Tyr Thr Trp Thr Arg Asp Ser
   45 50 55
   Gly Leu Val Leu Lys Thr Leu Val Asp Leu Phe Arg Asn Gly Asp Thr
   60 65 70
   Ser Leu Leu Ser Thr Ile Glu Asn Tyr Ile Ser Ala Gln Ala Ile Val
   75 80 85
   Gln Gly Ile Ser Asn Pro Ser Gly Asp Leu Ser Ser Gly Ala Gly Leu
   90 95 100
   Gly Glu Pro Lys Phe Asn Val Asp Glu Thr Ala Tyr Thr Gly Ser Trp
   105 110 115 120
   Gly Arg Pro Gln Arg Asp Gly Pro Ala Leu Arg Ala Thr Ala Met Ile
   125 130 135
   Gly Phe Gly Gln Trp Leu Leu Asp Asn Gly Tyr Thr Ser Thr Ala Thr
   140 145 150
   Asp Ile Val Trp Pro Leu Val Arg Asn Asp Leu Ser Tyr Val Ala Gln
   155 160 165
   Tyr Trp Asn Gln Thr Gly Tyr Asp Leu Trp Glu Glu Val Asn Gly Ser
   170 175 180
   Ser Phe Phe Thr Ile Ala Val Gln His Arg Ala Leu Val Glu Gly Ser
   185 190 195 200
   Ala Phe Ala Thr Ala Val Gly Ser Ser Cys Ser Trp Cys Asp Ser Gln
   205 210 215
   Ala Pro Glu Ile Leu Cys Tyr Leu Gln Ser Phe Trp Thr Gly Ser Phe
   220 225 230
   Ile Leu Ala Asn Phe Asp Ser Ser Arg Ser Gly Lys Asp Ala Asn Thr
   235 240 245
   Leu Leu Gly Ser Ile His Thr Phe Asp Pro Glu Ala Ala Cys Asp Asp
   250 255 260
   Ser Thr Phe Gln Pro Cys Ser Pro Arg Ala Leu Ala Asn His Lys Glu
   265 270 275 280
   Val Val Asp Ser Phe Arg Ser Ile Tyr Thr Leu Asn Asp Gly Leu Ser
   285 290 295
   Asp Ser Glu Ala Val Ala Val Gly Arg Tyr Pro Glu Asp Thr Tyr Tyr
   300 305 310
   Asn Gly Asn Pro Trp Phe Leu Cys Thr Leu Ala Ala Ala Glu Gln Leu
   315 320 325
   Tyr Asp Ala Leu Tyr Gln Trp Asp Lys Gln Gly Ser Leu Glu Val Thr
   330 335 340
   Asp Val Ser Leu Asp Phe Phe Lys Ala Leu Tyr Ser Asp Ala Ala Thr
   345 350 355 360
   Gly Thr Tyr Ser Ser Ser Ser Ser Thr Tyr Ser Ser Ile Val Asp Ala
   365 370 375
   Val Lys Thr Phe Ala Asp Gly Phe Val Ser Ile Val Glu Thr His Ala
   380 385 390
   Ala Ser Asn Gly Ser Met Ser Glu Gln Tyr Asp Lys Ser Asp Gly Glu
   395 400 405
   Gln Leu Ser Ala Arg Asp Leu Thr Trp Ser Tyr Ala Ala Leu Leu Thr
   410 415 420
   Ala Asn Asn Arg Arg Asn Ser Val Val Pro Ala Ser Trp Gly Glu Thr
   425 430 435 440
   Ser Ala Ser Ser Val Pro Gly Thr Cys Ala Ala Thr Ser Ala Ile Gly
   445 450 455
   Thr Tyr Ser Ser Val Thr Val Thr Ser Trp Pro Ser Ile Val Ala Thr
   460 465 470
   Gly Gly Thr Thr Thr Thr Ala Thr Pro Thr Gly Ser Gly Ser Val Thr
   475 480 485
   Ser Thr Ser Lys Thr Thr Ala Thr Ala Ser Lys Thr Ser Thr Thr Thr
   490 495 500
   Arg Ser Gly Met Ser Leu
   505 510
(2) INFORMATION FOR SEQ ID NO: 3:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ix) FEATURE:
      (A) NAME/KEY: misc-feature:
      (B) OTHER INFORMATION: /desc = "Primer 7258"
   (xi) SEQUENCE DESCRIPTION: SEQ ID NO: 3:
      gaatgacttg gttgacgcgt caccagtcac 20
2) INFORMATION FOR SEQ ID NO: 4:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ix) FEATURE:
      (A) NAME/KEY: misc-feature:
      (B) OTHER INFORMATION: /desc = "Primer 21401"
   (xi) SEQUENCE DESCRIPTION: SEQ ID NO: 4:
2) INFORMATION FOR SEQ ID NO: 5:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ix) FEATURE:
      (A) NAME/KEY: misc-feature:
      (B) OTHER INFORMATION: /desc = "Primer 107581"
   (xi) SEQUENCE DESCRIPTION: SEQ ID NO: 5:
      gcaacgaagc gcccgtggct cgtac 25
(2) INFORMATION FOR SEQ ID NO: 6:
   (i) SEQUENCE CHARACTERISTICS:
      (A) LENGTH: 109 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Primer FAMGII"
   (ix) FEATURE:
      (A) NAME/KEY: misc-feature
      (B) LOCATION: 30, 33, 42, 44, 47, 48, 51, 53, 56, 57, 58, 62,63,66, 69,71,72,73, 74, 75
      (D) : OTHER INFORMATION: /Note=
         1: A10,C90
         2: A6, T94
         3: A95,C5
         4:G95,C5
         5:G91,A3,T3,C3
         6:G95,A3,C2
         7:G3,A95,C2
         8:G92,A4,T4
         9: A3,T97
         10:G95,T5
         11 : G3,A97
         12 : G95,A2,C3
         13 : T5,C95
         14 : G88,A8,C4
         15:G7,A93
         16:G4,C96
         17:G4,A96
         18:G95,A2,C3
   (xi) SEQUENCE DESCRIPTION: SEQ ID NO: 6:
2) INFORMATION FOR SEQ ID NO: 7:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ix) FEATURE:
      (A) NAME/KEY: misc-feature:
      (B) OTHER INFORMATION: /desc = "Primer RAMG1"
   (xi) SEQUENCE DESCRIPTION: SEQ ID NO: 7:
      GATGGCAGTA CGAGCCACGG TCGCTTCG 28
(2) INFORMATION FOR SEQ ID NO: 8:
   (i) SEQUENCE CHARACTERISTICS:
      (A) LENGTH: 110 base pairs
      (B) TYPE: nucleic acid
      (C) STRANDEDNESS: single
      (D) TOPOLOGY: linear
   (ii) MOLECULE TYPE: other nucleic acid
   (ix) FEATURE:
      (A) NAME/KEY: misc-feature:
      (B) OTHER INFORMATION: /desc = "Primer FAMGIV"
   (ix) FEATURE:
      (A) NAME/KEY: misc-feature
         LOCATION : 22, 23, 24, 25, 26, 28, 31, 32, 34, 35,
         37, 40, 41, 43, 44, 46, 47, 49, 55, 56, 59, 60,
         61,62,67,68,70,71,74,77,79,80,85,86,87
      (D) : OTHER INFORMATION: /Note=
         1:G91,A3,T3,C3
         2:A13,C87
         3:A40,T60
         4:G3,A3,C94
         5:A6,T94
         6:G4,A4,T92
         7:G2,A96,C2
         8:G93,A3.5,C3.5
         9:G87,A8,C5
         10:A84,C16
         11:G93,T7
         12:G92,A5,T3
         13:A3,C97
         14:G3,A97
         15:G2,A2,T4,C92
         16:G93,A7
         17:G93,C7
         18:A90,T10
         19:G4,A96
         20:G95,A5
         21:G96,A4
         22:G3,C97
         23:G2,A1,T95,C2
         24:A3,C97
         25:G95,A3,C2
         26:G2,A96,C2
         27:A5,C95
         28:A95,T5
         29:G2,A98
         30:G94,A4,C2
         31:G94,A3,T1,C2
         32:A4,T96
         33:A20,C80
   (xi) SEQUENCE DESCRIPTION: SEQ ID NO: 8:
2) INFORMATION FOR SEQ ID NO: 9:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ix) FEATURE:
      (A) NAME/KEY: misc-feature:
      (B) OTHER INFORMATION: /desc = "Primer RAMGVI"
   (xi) SEQUENCE DESCRIPTION: SEQ ID NO: 9:
      CTTGAAGAAG TCCAGCGACA C 21
2) INFORMATION FOR SEQ ID NO: 10:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ix) FEATURE:
      (A) NAME/KEY: misc-feature:
      (B) OTHER INFORMATION: /desc = "Primer FG2"
   (xi) SEQUENCE DESCRIPTION: SEQ ID NO: 10:
      CATCCCCAGG ATCCTTACTC AGCAATG 27
2) INFORMATION FOR SEQ ID NO: 11:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ix) FEATURE:
      (A) NAME/KEY: misc-feature:
      (B) OTHER INFORMATION: /desc = "Primer RG2"
   (xi) SEQUENCE DESCRIPTION: SEQ ID NO: 11:
      CTCAAACGAC TCACCAGCCT CTAGAGT 27

<210> 12
   <211> 2602
   <212> DNA
   <213> Aspergillus niger
<220>
<221> sig_peptide
   <222> (270) ... (323)
<221> mat_peptide
   <222> (342)...(2438)
<221> intron
   <222> (484)...(558)
<221> intron
   <222> (846) ... (900)
<221> intron
   <222> (998)...(1058)
<221> intron
   <222> (1697) ... (1754)
<400> 12
   ttcgtcgcct aatgtctcgt ccgttcacaa actgaagagc ttgaagtggc gagatgtctc 60
   tgcaggaatt caagctagat gctaagcgat attgcatggc aatatgtgtt gatgcatgtg 120
   cttcttcctt cagcttcccc tcgtgcgagt gaggtttggc tataaattga agtggttggt 180
   cggggttccg tgaggggctg aagtgcttcc tcccttttag gcgcaactga gagcctgagc 240
   ttcatcccca gcatcattac acctcagcaa tgtcgttccg atctctactc gccctgagcg 300
   gcctcgtctg cacagggttg gcaaatgtga tttccaagcg cgcgaccttg gattcatggt 360
   tgagcaacga agcgaccgtg gctcgtactg ccatcctgaa taacatcggg gcggacggtg 420
   cttgggtgtc gggcgcggac tctggcattg tcgttgctag tcccagcacg gataacccgg 480
   actgtatgtt tcgagctcag atttagtatg agtgtgtcat tgattgattg atgctgactg 540
   gcgtgtcgtt tgttgtagac ttctacacct ggactcgcga ctctggtctc gtcctcaaga 600
   ccctcgtcga tctcttccga aatggagata ccagtctcct ctccaccatt gagaactaca 660
   tctccgccca ggcaattgtc cagggtatca gtaacccctc tggtgatctg tccagcggcg 720
   ctggtctcgg tgaacccaag ttcaatgtcg atgagactgc ctacactggt tcttggggac 780
   ggccgcagcg agatggtccg gctctgagag caactgctat gatcggcttc gggcagtggc 840
   tgcttgtatg ttctccaccc ccttgcgtct gatctgtgac atatgtagct gactggtcag 900
   gacaatggct acaccagcac cgcaacggac attgtttggc ccctcgttag gaacgacctg 960
   tcgtatgtgg ctcaatactg gaaccagaca ggatatggtg tgtttgtttt attttaaatt 1020
   tccaaagatg cgccagcaga gctaacccgc gatcgcagat ctctgggaag aagtcaatgg 1080
   ctcgtctttc tttacgattg ctgtgcaaca ccgcgccctt gtcgaaggta gtgccttcgc 1140
   gacggccgtc ggctcgtcct gctcctggtg tgattctcag gcacccgaaa ttctctgcta 1200
   cctgcagtcc ttctggaccg gcagcttcat tctggccaac ttcgatagca gccgttccgg 1260
   caaggacgca aacaccctcc tgggaagcat ccacaccttt gatcctgagg ccgcatgcga 1320
   cgactccacc ttccagccct gctccccgcg cgcgctcgcc aaccacaagg aggttgtaga 1380
   ctctttccgc tcaatctata ccctcaacga tggtctcagt gacagcgagg ctgttgcggt 1440
   gggtcggtac cctgaggaca cgtactacaa cggcaacccg tggttcctgt gcaccttggc 1500
   tgccgcagag cagttgtacg atgctctata ccagtgggac aagcaggggt cgttggaggt 1560
   cacagatgtg tcgctggact tcttcaaggc actgtacagc gatgctgcta ctggcaccta 1620
   ctcttcgtcc agttcgactt atagtagcat tgtagatgcc gtgaagactt tcgccgatgg 1680
   cttcgtctct attgtggtaa gtctacgcta gacaagcgct catgttgaca gagggtgcgt 1740
   actaacagaa gtaggaaact cacgccgcaa gcaacggctc catgtccgag caatacgaca 1800
   agtctgatgg cgagcagctt tccgctcgcg acctgacctg gtcttatgct gctctgctga 1860
   ccgccaacaa ccgtcgtaac tccgtcgtgc ctgcttcttg gggcgagacc tctgccagca 1920
   gcgtgcccgg cacctgtgcg gccacatctg ccattggtac ctacagcagt gtgactgtca 1980
   cctcgtggcc gagtatcgtg gctactggcg gcaccactac gacggctacc cccactggat 2040
   ccggcagcgt gacctcgacc agcaagacca ccgcgactgc tagcaagacc agcaccagta 2100
   cgtcatcaac ctcctgtacc actcccaccg ccgtggctgt gactttcgat ctgacagcta 2160
   ccaccaccta cggcgagaac atctacctgg tcggatcgat ctctcagctg ggtgactggg 2220
   aaaccagcga cggcatagct ctgagtgctg acaagtacac ttccagcgac ccgctctggt 2280
   atgtcactgt gactctgccg gctggtgagt cgtttgagta caagtttatc cgcattgaga 2340
   gcgatgactc cgtggagtgg gagagtgatc ccaaccgaga atacaccgtt cctcaggcgt 2400
   gcggaacgtc gaccgcgacg gtgactgaca cctggcggtg acaatcaatc catttcgcta 2460
   tagttaaagg atggggatga gggcaattgg ttatatgatc atgtatgtag tgggtgtgca 2520
   taatagtagt gaaatggaag ccaagtcatg tgattgtaat cgaccgacgg aattgaggat 2580
   atccggaaat acagacaccg gg 2602
<210> SEQ ID NO:13
   <211> LENGTH: 640
   <212> TYPE: PRT
   <213> ORGANISM: Aspergillus niger
<400> SEQ ID NO: 13
<210> SEQ ID NO:14
   <211> LENGTH: 43
   <212> TYPE: DNA
   <213> ORGANISM:Artificial Sequence
<220> FEATURE:
   <223> OTHER INFORMATION: primer HK1-T2X
<400> SEQ ID NO:14
   atgtgatttc caagcgcgcg vnnttggatt catggttgag caa 43
<210> SEQ ID NO:15
   <211> LENGTH: 43
   <212> TYPE: DNA
   <213> ORGANISM:Artificial Sequence
<220> FEATURE:
   <223> OTHER INFORMATION: primer Hk1-N9X
<400> SEQ ID NO:15
   ccttggattc atggttgagc vnngaagcga ccgtggctcg tac 43
<210> SEQ ID NO:16
   <211> LENGTH: 43
   <212> TYPE: DNA
   <213> ORGANISM:Artificial Sequence
<220> FEATURE:
   <223> OTHER INFORMATION: primer Hk1-A11X
<400> SEQ ID NO:16
   attcatggtt gagcaacgaa vnnaccgtgg ctcgtactgc cat 43
<210> SEQ ID NO:17
   <211> LENGTH: 43
   <212> TYPE: DNA
   <213> ORGANISM:Artificial Sequence
<220> FEATURE:
   <223> OTHER INFORMATION: primer Hk1-L66X
<400> SEQ ID NO:17
   tcctcaagac cctcgtcgat vnnttccgaa atggagatac cag 43
<210> SEQ ID NO:18
   <211> LENGTH: 43
   <212> TYPE: DNA
   <213> ORGANISM:Artificial Sequence
<220> FEATURE:
   <223> OTHER INFORMATION: primer Hk1-S386X
<400> SEQ ID NO:18
   ctttcgccga tggcttcgtc vnnattgtgg aaactcacgc cgc 43
<210> SEQ ID NO:19
   <211> LENGTH: 43
   <212> TYPE: DNA
   <213> ORGANISM:Artificial Sequence
<220> FEATURE:
   <223> OTHER INFORMATION: primer Hk1-E389X
<400> SEQ ID NO:19
   atggcttcgt ctctattgtg vnnactcacg ccgcaagcaa cgg 43
<210> SEQ ID NO:20
   <211> LENGTH: 43
   <212> TYPE: DNA
   <213> ORGANISM:Artificial Sequence
<220> FEATURE:
   <223> OTHER INFORMATION: primer Hk1-T390X
<400> SEQ ID NO:20
   GCTTCGTCTC TATTGTGGAA VNNCACGCCG CAAGCAACGG CTC 43
<210> SEQ ID NO:21
   <211> LENGTH: 43
   <212> TYPE: DNA
   <213> ORGANISM:Artificial Sequence
<220> FEATURE:
   <223> OTHER INFORMATION: primer Hk1-A393X
<400> SEQ ID NO:21
   ctattgtgga aactcacgcc vnnagcaacg gctccatgtc cga 43
<210> SEQ ID NO:22
   <211> LENGTH: 43
   <212> TYPE: DNA
   <213> ORGANISM:Artificial Sequence
<220> FEATURE:
   <223> OTHER INFORMATION: primer Hk1-S394X
<400> SEQ ID NO:22
   ttgtggaaac tcacgccgca vnnaacggct ccatgtccga gca 43
<210> SEQ ID NO:23
   <211> LENGTH: 43
   <212> TYPE: DNA
   <213> ORGANISM:Artificial Sequence
<220> FEATURE:
   <223> OTHER INFORMATION: primer Hk1-N395X
<400> SEQ ID NO:23
   tggaaactca cgccgcaagc vnnggctcca tgtccgagca ata 43
<210> SEQ ID NO:24
   <211> LENGTH: 43
   <212> TYPE: DNA
   <213> ORGANISM:Artificial Sequence
<220> FEATURE:
   <223> OTHER INFORMATION: primer Hk1-G296X
<400> SEQ ID NO:24
   aaactcacgc cgcaagcaac vnntccatgt ccgagcaata cga 43
<210> SEQ ID NO:25
   <211> LENGTH: 43
   <212> TYPE: DNA
   <213> ORGANISM:Artificial Sequence
<220> FEATURE:
   <223> OTHER INFORMATION: primer Hk1-K404X
<400> SEQ ID NO:25
   ccatgtccga gcaatacgac vnntctgatg gcgagcagct ttc 43
<210> SEQ ID NO:26
   <211> LENGTH: 43
   <212> TYPE: DNA
   <213> ORGANISM:Artificial Sequence
<220> FEATURE:
   <223> OTHER INFORMATION: primer Hk1-D406X
<400> SEQ ID NO:26
   ccgagcaata cgacaagtct vnnggcgagc agctttccgc tcg 43
<210> SEQ ID NO:27
   <211> LENGTH: 43
   <212> TYPE: DNA
   <213> ORGANISM:Artificial Sequence
<220> FEATURE:
   <223> OTHER INFORMATION: primer Hk1-E408X
<400> SEQ ID NO:27
   aatacgacaa gtctgatggc vnncagcttt ccgctcgcga cct 43
<210> SEQ ID NO:28
   <211> LENGTH: 42
   <212> TYPE: DNA
   <213> ORGANISM:Artificial Sequence
<220> FEATURE:
   <223> OTHER INFORMATION: primer Hk1-L410X
<400> SEQ ID NO:28
   acaagtctga tggcgagcag vnntccgctc gcgacctgac ct 42
<210> SEQ ID NO:29
   <211> LENGTH: 43
   <212> TYPE: DNA
   <213> ORGANISM:Artificial Sequence
<220> FEATURE:
   <223> OTHER INFORMATION: primer Hk1-L423X
<400> SEQ ID NO:29
   cctggtctta tgctgctctg vnnaccgcca acaaccgtcg taa 43
<210> SEQ ID NO : 30
   <211> LENGTH: 44
   <212> TYPE: DNA
   <213> ORGANISM:Artificial Sequence
<220> FEATURE:
   <223> OTHER INFORMATION: primer Hk1-N426X
<400> SEQ ID NO:30
   atgctgctct gctgaccgcc vnnaaccgtc gtaactccgt cgtg 44
<210> SEQ ID NO:31
   <211> LENGTH: 44
   <212> TYPE: DNA
   <213> ORGANISM:Artificial Sequence
<220> FEATURE:
   <223> OTHER INFORMATION: primer Hk1-N427X
<400> SEQ ID NO:31
   CTGCTCTGCT GACCGCCAAC VNNCGTCGTA ACTCCGTCGT GCCT 44
<210> SEQ ID NO:32
   <211> LENGTH: 46
   <212> TYPE: DNA
   <213> ORGANISM:Artificial Sequence
<220> FEATURE:
   <223> OTHER INFORMATION: primer Hk1-Y402X
<400> SEQ ID NO:32
   ACGGCTCCAT GTCCGAGCAA NNCGACAAGT CTGATGGCGA GCAGCT 46
<210> SEQ ID NO:33
   <211> LENGTH: 41
   <212> TYPE: DNA
   <213> ORGANISM:Artificial Sequence
<220> FEATURE:
   <223> OTHER INFORMATION: primer Hk2-L234X-sense
<400> SEQ ID NO:33
   ctggaccggc agcttcattn nkgccaactt cgatagcagc c 41
<210> SEQ ID NO:34
   <211> LENGTH: 42
   <212> TYPE: DNA
   <213> ORGANISM:Artificial Sequence
<220> FEATURE:
   <223> OTHER INFORMATION: primer Hk2-A235S-antisense
<400> SEQ ID NO:34
   gaacggctgc tatcgaagtt agacagaatg aagctgccgg tc 42
<210> SEQ ID NO:35
   <211> LENGTH: 41
   <212> TYPE: DNA
   <213> ORGANISM:Artificial Sequence
<220> FEATURE:
   <223> OTHER INFORMATION: primer Hk2-NF237X-sense
<400> SEQ ID NO:35
   cagcttcatt ctggccaacn atgatagcag ccgttccggc a 41
<210> SEQ ID NO : 36
   <211> LENGTH: 42
   <212> TYPE: DNA
   <213> ORGANISM:Artificial Sequence
<220> FEATURE:
   <223> OTHER INFORMATION: primer Hk2-D238T-antisense
<400> SEQ ID NO:36
   ccttgccgga acggctgcta gtgaagttgg ccagaatgaa gc 42
<210> SEQ ID NO:37
   <211> LENGTH: 42
   <212> TYPE: DNA
   <213> ORGANISM:Artificial Sequence
<220> FEATURE:
   <223> OTHER INFORMATION: primer Hk2-D238S-antisense
<400> SEQ ID NO:37
   ccttgccgga acggctgcta gagaagttgg ccagaatgaa gc 42
<210> SEQ ID NO:38
   <211> LENGTH: 42
   <212> TYPE: DNA
   <213> ORGANISM:Artificial Sequence
<220> FEATURE:
   <223> OTHER INFORMATION: primer Hk2-S239X-sense
<400> SEQ ID NO:38
   tcattctggc caacttcgat nncagccgtt ccggcaagga cg 42
<210> SEQ ID NO:39
   <211> LENGTH: 42
   <212> TYPE: DNA
   <213> ORGANISM:Artificial Sequence
<220> FEATURE:
   <223> OTHER INFORMATION: primer Hk2-S240G-antisense
<400> SEQ ID NO:39
   ttgcgtcctt gccggaacga ccgctatcga agttggccag aa 42
<210> SEQ ID NO;40
   <211> LENGTH: 41
   <212> TYPE: DNA
   <213> ORGANISM:Artificial Sequence
<220> FEATURE:
   <223> OTHER INFORMATION: primer Hk2-S242X-antisense
<400> SEQ ID NO:40
   gggtgtttgc gtccttgcca knacggctgc tatcgaagtt g 41
<210> SEQ ID NO:41
   <211> LENGTH: 41
   <212> TYPE: DNA
   <213> ORGANISM:Artificial Sequence
<220> FEATURE:
   <223> OTHER INFORMATION: primer Hk2-G243X-antisense
<400> SEQ ID NO:41
   ggagggtgtt tgcgtcctta knggaacggc tgctatcgaa g 41
<210> SEQ ID NO:42
   <211> LENGTH: 41
   <212> TYPE: DNA
   <213> ORGANISM:Artificial Sequence
<220> FEATURE:
   <223> OTHER INFORMATION: primer Hk2-K244R-sense
<400> SEQ ID NO:42
   cgatagcagc cgttccggca gagacgcaaa caccctcctg g 41
<210> SEQ ID NO:43
   <211> LENGTH: 41
   <212> TYPE: DNA
   <213> ORGANISM:Artificial Sequence
<220> FEATURE:
   <223> OTHER INFORMATION: primer Hk2-T310V-antisense
<400> SEQ ID NO:43
   acgggttgcc gttgtagtaa acgtcctcag ggtaccgacc c 41
<210> SEQ ID NO:44
   <211> LENGTH: 41
   <212> TYPE: DNA
   <213> ORGANISM:Artificial Sequence
<220> FEATURE:
   <223> OTHER INFORMATION: primer Hk2-T310S-antisemse
<400> SEQ ID NO:44
   acgggttgcc gttgtagtaa gagtcctcag ggtaccgacc c 41
<210> SEQ ID NO:45
   <211> LENGTH: 41
   <212> TYPE: DNA
   <213> ORGANISM:Artificial Sequence
<220> FEATURE:
   <223> OTHER INFORMATION: primer Hk2-Y311N-sense
<400> SEQ ID NO:45
   tcggtaccct gaggacacga attacaacgg caacccgtgg t 41
<210> SEQ ID NO:46
   <211> LENGTH: 41
   <212> TYPE: DNA
   <213> ORGANISM:Artificial Sequence
<220> FEATURE:
   <223> OTHER INFORMATION: primer Hk2-Y312Q-antisense
<400> SEQ ID NO:46
   ggaaccacgg gttgccgttt tggtacgtgt cctcagggta c 41
<210> SEQ ID NO:47.
   <211> LENGTH: 41
   <212> TYPE: DNA
   <213> ORGANISM:Artificial Sequence
<220> FEATURE:
   <223> OTHER INFORMATION: primer Hk2-Y312N-antisese
<400> SEQ ID NO:47
   ggaaccacgg gttgccgtta ttgtacgtgt cctcagggta c 41
<210> SEQ ID NO:48
   <211> LENGTH: 41
   <212> TYPE: DNA
   <213> ORGANISM:Artificial Sequence
<220> FEATURE:
   <223> OTHER INFORMATION: primer Hk2-N313T-sense
<400> SEQ ID NO:48
   ccctgaggac acgtactaca ctggcaaccc gtggttcctg t 41
<210> SEQ ID NO:49
   <211> LENGTH: 41
   <212> TYPE: DNA
   <213> ORGANISM:Artificial Sequence
<220> FEATURE:
   <223> OTHER INFORMATION: primer Hk2-N313S-sense
<400> SEQ ID NO:49
   ccctgaggac acgtactact ctggcaaccc gtggttcctg t 41
<210> SEQ ID NO:50
   <211> LENGTH: 41
   <212> TYPE: DNA
   <213> ORGANISM:Artificial Sequence
<220> FEATURE:
   <223> OTHER INFORMATION: primer Hk2-N313G-sense
<400> SEQ ID NO:50
   ccctgaggac acgtactacg gtggcaaccc gtggttcctg t 41
<210> SEQ ID NO:51
   <211> LENGTH: 41
   <212> TYPE: DNA
   <213> ORGANISM:Artificial Sequence
<220> FEATURE:
   <223> OTHER INFORMATION: primer Hk2-N315Q-antisense
<400> SEQ ID NO : 51
   aggtgcacag gaaccacggt tggccgttgt agtacgtgtc c 41
<210> SEQ ID NO:52
   <211> LENGTH: 41
   <212> TYPE: DNA
   <213> ORGANISM:Artificial Sequence
<220> FEATURE:
   <223> OTHER INFORMATION: primer Hk2-N315E-antisense
<400> SEQ ID NO : 52
   aggtgcacag gaaccacggt tcgccgttgt agtacgtgtc c 41
<210> SEQ ID NO:53
   <211> LENGTH: 41
   <212> TYPE: DNA
   <213> ORGANISM:Artificial Sequence
<220> FEATURE:
   <223> OTHER INFORMATION: primer Hk2-N315R-antisense
<400> SEQ ID NO:53
   aggtgcacag gaaccacggt ctgccgttgt agtacgtgtc c 41
<210> SEQ ID NO:54
   <211> LENGTH: 41 <212> TYPE: DNA
   <213> ORGANISM:Artificial Sequence
<220> FEATURE:
   <223> OTHER INFORMATION: primer Hk2-F318Y-antisense
<400> SEQ ID NO:54
   cggcagccaa ggtgcacaga taccacgggt tgccgttgta g 41
<210> SEQ ID NO:55
   <211> LENGTH: 41
   <212> TYPE: DNA
   <213> ORGANISM:Artificial Sequence
<220> FEATURE:
   <223> OTHER INFORMATION: primer Hk2-Q409P-sense
<400> SEQ ID NO:55
   cgacaagtct gatggcgagc cactttccgc tcgcgacctg a 41
<210> SEQ ID NO:56
   <211> LENGTH: 41
   <212> TYPE: DNA
   <213> ORGANISM:Artificial Sequence
<220> FEATURE:
   <223> OTHER INFORMATION: primer Hk3-D336X-sense
<400> SEQ ID NO:56
   cgatgctcta taccagtggn nkaagcaggg gtcgttggag g 41
<210> SEQ ID NO:57
   <211> LENGTH: 41
   <212> TYPE: DNA
   <213> ORGANISM:Artificial Sequence
<220> FEATURE:
   <223> OTHER INFORMATION: primer Hk3-K337X-sense
<400> SEQ ID NO:57
   tgctctatac cagtgggacn nkcaggggtc gttggaggtc a 41
<210> SEQ ID NO:58
   <211> LENGTH: 41
   <212> TYPE: DNA
   <213> ORGANISM:Artificial Sequence
<220> FEATURE:
   <223> OTHER INFORMATION: primer Hk3-Q338X-antisense
<400> SEQ ID NO:58
   ctgtgacctc caacgacccg nncttgtccc actggtatag a 41
<210> SEQ ID NO:59
   <211> LENGTH: 41
   <212> TYPE: DNA
   <213> ORGANISM:Artificial Sequence
<220> FEATURE:
   <223> OTHER INFORMATION: primer Hk3-G339X-sense
<400> SEQ ID NO:59
   ataccagtgg gacaagcagn cutcgttgga ggtcacagat g 41
<210> SEQ ID NO:60
   <211> LENGTH: 41
   <212> TYPE: DNA
   <213> ORGANISM:Artificial Sequence
<220> FEATURE:
   <223> OTHER INFORMATION: primer Hk3-S34OX-antisense
<400> SEQ ID NO:60
   acacatctgt gacctccaaa ntcccctgct tgtcccactg g 41
<210> SEQ ID NO:61
   <211> LENGTH: 41
   <212> TYPE: DNA
   <213> ORGANISM:Artificial Sequence
<220> FEATURE:
   <223> OTHER INFORMATION: primer Hk3-S340C-antisense
<400> SEQ ID NO:61
   acacatctgt gacctccaaa nccccctgct tgtcccactg g 41
<210> SEQ ID NO:62
   <211> LENGTH: 41
   <212> TYPE: DNA
   <213> ORGANISM:Artificial Sequence
<220> FEATURE:
   <223> OTHER INFORMATION: primer Hk3-L341X-sense
<400> SEQ ID NO:62
   gtgggacaag caggggtcgn uugaggtcac agatgtgtcg c 41
<210> SEQ ID NO:63
   <211> LENGTH: 41
   <212> TYPE: DNA
   <213> ORGANISM:Artificial Sequence
<220> FEATURE:
   <223> OTHER INFORMATION: primer Hk3-K352Q-sense
<400> SEQ ID NO:63
   tgtgtcgctg gacttcttcc aagcactgta cagcgatgct g 41
<210> SEQ ID NO:64
   <211> LENGTH: 41
   <212> TYPE: DNA
   <213> ORGANISM:Artificial Sequence
<220> FEATURE:
   <223> OTHER INFORMATION: primer Hk3-K352R-sense
<400> SEQ ID NO:64
   tgtgtcgctg gacttcttca gagcactgta cagcgatgct g 41
<210> SEQ ID NO:65
   <211> LENGTH: 41
   <212> TYPE: DNA
   <213> ORGANISM:Artificial Sequence
<220> FEATURE:
   <223> OTHER INFORMATION: primer Hk3-A352D-antisense
<400> SEQ ID NO:65
   tagcagcatc gctgtacaga tccttgaaga agtccagcga c 41
<210> SEQ ID NO:66
   <211> LENGTH: 41
   <212> TYPE: DNA
   <213> ORGANISM:Artificial Sequence
<220> FEATURE:
   <223> OTHER INFORMATION: primer Hk3-A353S-antisense
<400> SEQ ID NO:66
   tagcagcatc gctgtacaga gacttgaaga agtccagcga c 41
<210> SEQ ID NO:67
   <211> LENGTH: 41
   <212> TYPE: DNA
   <213> ORGANISM:Artificial Sequence
<220> FEATURE:
   <223> OTHER INFORMATION: primer Hk3-S356P-sense
<400> SEQ ID NO:67
   acttcttcaag gcactgtacc cagatgctgc tactggcacc t 41
<210> SEQ ID NO:68
   <211> LENGTH: 43
   <212> TYPE: DNA
   <213> ORGANISM:Artificial Sequence
<220> FEATURE:
   <223> OTHER INFORMATION: primer Hk3-S356N-sense
<400> SEQ ID NO:68
   acttcttcaa ggcactgtac aaugatgctg ctactggcac cta 43
<210> SEQ ID NO:69
   <211> LENGTH: 43
   <212> TYPE: DNA
   <213> ORGANISM:Artificial Sequence
<220> FEATURE:
   <223> OTHER INFORMATION: primer Hk3-S356X-sense
<400> SEQ ID NO:69
   acttcttcaa ggcactgtac gaugatgctg ctactggcac cta 43
<210> SEQ ID NO:70
   <211> LENGTH: 43
   <212> TYPE: DNA
   <213> ORGANISM:Artificial Sequence
<220> FEATURE:
   <223> OTHER INFORMATION: primer Hk3-D357S-antisense
<400> SEQ ID NO:70
   gagtaggtgc cagtagcagc agagctgtac agtgccttga aga 43
<210> SEQ ID NO:71
   <211> LENGTH: 41
   <212> TYPE: DNA
   <213> ORGANISM:Artificial Sequence
<220> FEATURE:
   <223> OTHER INFORMATION: primer Hk3-A359S-sense
<400> SEQ ID NO : 71
   ggcactgtac agcgatgctt ctactggcac ctactcttcg t 41
<210> SEQ ID NO:72
   <211> LENGTH: 41
   <212> TYPE: DNA
   <213> ORGANISM:Artificial Sequence
<220> FEATURE:
   <223> OTHER INFORMATION: primer Hk3-T360V-antisense
<400> SEQ ID NO:72
   tggacgaaga gtaggtgcca acagcagcat cgctgtacag t 41
<210> SEQ ID NO:73
   <211> LENGTH: 43
   <212> TYPE: DNA
   <213> ORGANISM:Artificial Sequence
<220> FEATURE:
   <223> OTHER INFORMATION: primer Hk3-G361X-sense
<400> SEQ ID NO:73
   tgtacagcga tgctgctact nctacctact cttcgtccag ttc 43
<210> SEQ ID NO:74
   <211> LENGTH: 42
   <212> TYPE: DNA
   <213> ORGANISM:Artificial Sequence
<220> FEATURE:
   <223> OTHER INFORMATION: primer Hk3-T362R-antisense
<400> SEQ ID NO:74
   gtcgaactgg acgaagagta tctgccagta gcagcatcgc tg 42
<210> SEQ ID NO:75
   <211> LENGTH: 42
   <212> TYPE: DNA
   <213> ORGANISM:Artificial Sequence
<220> FEATURE:
   <223> OTHER INFORMATION: primer Hk3-S364X-sense
<400> SEQ ID NO:75
   tgctgctact ggcacctacn nktcgtccag ttcgacttat ag 42
<210> SEQ ID NO:76
   <211> LENGTH: 42
   <212> TYPE: DNA
   <213> ORGANISM:Artificial Sequence
<220> FEATURE:
   <223> OTHER INFORMATION: primer Hk3-S365X-sense
<400> SEQ ID NO:76
   tgctactggc acctactctn nktccagttc gacttatagt ag 42
<210> SEQ ID NO:77
   <211> LENGTH: 42
   <212> TYPE: DNA
   <213> ORGANISM:Artificial Sequence
<220> FEATURE:
   <223> OTHER INFORMATION: primer Hk3-S366T-antisense
<400> SEQ ID NO : 77
   atgctactat aagtcgaact agtcgaagag taggtgccag ta 42
<210> SEQ ID NO:78
   <211> LENGTH: 42
   <212> TYPE: DNA
   <213> ORGANISM:Artificial Sequence
<220> FEATURE:
   <223> OTHER INFORMATION: primer Hk3-S368X-antisense
<400> SEQ ID NO:78
   tctacaatgc tactataagt agnactggac gaagagtagg tg 42
<210> SEQ ID NO:79
   <211> LENGTH: 43
   <212> TYPE: DNA
   <213> ORGANISM:Artificial Sequence
<220> FEATURE:
   <223> OTHER INFORMATION: primer Hk3-T369X-sense
<400> SEQ ID NO:79
   ctactcttcg tccagttcgn nktatagtag cattgtagat gcc 43
<210> SEQ ID NO:80
   <211> LENGTH: 43
   <212> TYPE: DNA
   <213> ORGANISM:Artificial Sequence
<220> FEATURE:
   <223> OTHER INFORMATION: primer Hk3-S371X-antisense
<400> SEQ ID NO:80
   ttcacggcat ctacaatgct atnataagtc gaactggacg aag 43
<210> SEQ ID NO:81
   <211> LENGTH: 43
   <212> TYPE: DNA
   <213> ORGANISM:Artificial Sequence
<220> FEATURE:
   <223> OTHER INFORMATION: primer Hk3-S372X-sense
<400> SEQ ID NO:81
   cgtccagttc gacttatagt nntattgtag atgccgtgaa gac 43

## Claims

1. A variant of a parent glucoamylase having glucoamylase activity and improved thermostability over the parent glucoamylase, the variant having the amino acid sequence of SEQ ID NO:2 and comprising one or more mutation(s) in the following position(s) in the amino acid sequence shown in SEQ ID NO:2 E408R+S386N, T2Q+A11P+S394R, A11 E+E408R, T2M+N9A+T390R+D406N+L410R, T2R+S386R+A393R, A393R+L410R, A1V+L66R+Y402F+N427S+S486G, T2E+T379A+S386K+A393R, V59A+A393R+T490A and N-terminal extension consisting of PLASD, Y402F+S411V, S119P+Y402F+S411V, S119P+Y312Q+Y402F+T416H,
or the variant having the one or more mutation(s) in a corresponding position in a homologous glucoamylase which displays a degree of identity of at least 60% with the amino acid sequences shown in SEQ ID NO:2.

2. A variant of a parent glucoamylase having glucoamylase activity and improved thermostability over the parent glucoamylase, the variant having the amino acid sequence of SEQ ID NO:2 and comprising the mutation A393R,
or the variant having the mutation in a corresponding position in a homologous glucoamylase which displays a degree of identity of at least 70% with the amino acid sequences shown in SEQ ID NO:2.

3. A variant of a parent glucoamylase having glucoamylase activity and improved thermostability over the parent glucoamylase, the variant having the amino acid sequence of SEQ ID NO:2 and comprising the mutation Y402F,
or the variant having the mutation in a corresponding position in a homologous glucoamylase which displays a degree of identity of at least 95% with the amino acid sequences shown in SEQ ID NO:2.

4. The variant according to any of Claims 1-3, wherein the parent homologous glucoamylase is the Aspergillus niger G1 glucoamylase.

5. The variant according to any of Claims 1-4, wherein the glucoamylase is a truncated glucoamylase, in particular in the C-terminal.

6. A DNA construct comprising a DNA sequence encoding a glucoamylase variant according to any one of Claims 1-5.

7. A recombinant expression vector which carries a DNA construct according to Claim 6.

8. A cell which is transformed with a DNA construct according to Claim 6 or a vector according to Claim 7.

9. A cell according to Claim 8, which is a microorganism, such as a bacterium or a fungus.

10. The cell according to Claim 9, which is a protease deficient Aspergillus oryzae or Aspergillus niger.

11. A process for converting starch or partially hydrolyzed starch into a syrup containing dextrose, said process including the step of saccharifying starch hydrolyzate in the presence of a glucoamylase variant according to any of Claims 1-5.

12. The process of Claim 11, wherein the dosage of glucoamylase is present in the range from 0.05 to 0.5 AGU per gram of dry solids.

13. The process of any of Claims 11 or 12, comprising saccharification of a starch hydrolyzate of at least 30 percent by weight of dry solids.

14. The process of any of Claims 11-13, wherein the saccharification is conducted in the presence of a debranching enzyme selected from the group of pullulanase and isoamylase, preferably a pullulanase derived from Bacillus acidopullulyticus or Bacillus deramificans or an isoamylase derived from Pseudomonas amyloderamosa.

15. The process of any of Claims 11-14, wherein the saccharification is conducted at a pH of 3 to 5.5 and at a temperature of 60-80°C, preferably 63-75°C, for 24 to 72 hours, preferably for 36-48 hours at a pH from 4 to 4.5.

16. A method of saccharifying a liquefied starch solution, which method comprises
(i) a saccharification step during which step one or more enzymatic saccharification stages takes place, and
the subsequent step of
(ii) one or more high temperature membrane separation steps
wherein the enzymatic saccharification is carried out using a glucoamylase variant according to any of Claims 1 to 5.

17. Use of a glucoamylase variant according to any of Claims 1-5 in a starch conversion process.

18. Use of a glucoamylase variant according to any of Claims 1-5 in a continuous starch conversion process.

19. Use according to Claim 18, wherein the continuous starch conversion process include a continuous saccharification process according to Claim 16.

20. Use of a glucoamylase variant according to any of Claims 1-5 in a process for producing oligosaccharides.

21. Use of a glucoamylase variant according to any of Claims 1-5 in a process for producing specialty syrups.

22. Use of a glucoamylase variant according to any one of Claims 1-5 in a process for producing ethanol for fuel.

23. Use of a glucoamylase variant according to any one of Claims 1-5 in a process for producing a beverage.

24. Use of a glucoamylase variant according to any one of Claims 1-5 in a fermentation process for producing organic compounds, such as citric acid, ascorbic acid, lysine, glutamic acid.

25. A method for improving the thermostability of a parent glucoamylase having the amino acid sequence of SEQ ID NO:2 by making a mutation in one or more of the following position(s) in the amino acid sequence shown in SEQ ID NO: 2: E408R+S386N, T2Q+A11P+S394R, A11E+E408R, T2M+N9A+T390R+D406N+L410R, A393R, T2R+S386R+A393R, A393R+L410R, A1V+L66R+Y402F+N427S+S486G, T2E+T379A+S386K+A393R, Y402F, V59A+A393R+T490A and N-terminal extension consisting of PLASD, Y402F+8411V, S119P+Y402F+S411V, S119P+Y312Q+Y402F+T416H,
or for improving the thermostability of a parent glucoamylase which displays a degree of identity of at least 60% with the amino acid sequences shown in SEQ ID NO:2 by making the one or more mutations in a corresponding position in the homologous glucoamylase.

## Patentansprüche

1. Variante einer Eltern-Glucoamylase mit Glucoamylaseaktivität und verbesserter Wärmestabilität gegenüber der Eltern-Glucoamylase, wobei die Variante die Aminosäuresequenz von SEQ ID NO:2 aufweist und eine oder mehrere Mutation(en) in den folgenden Position(en) in der in SEQ ID NO:2 gezeigten Aminosäuresequenz einschließt E408R+S386N, T2Q+A11P+S394R, A11E+E408R, T2M+N9A+T390R+D406N+L410R, T2R+S386R+A393R, A393R+L410R, A1V+L66R+Y402F+N427S+S486G, T2E+T379A+S386K+A393R, V59A+A393R+T490A und eine N-terminale Extension, bestehend aus PLASD, Y402F+S411V, S119P+Y402F+S411V, S119P+Y312Q+Y402F+T416H,
oder die Variante mit der einen oder den mehreren Mutation(en) in einer entsprechenden Position in einer homologen Glucoamylase, die einen Identitätsgrad von mindestens 60 % mit den in SEQ ID NO:2 gezeigten Aminosäuresequenzen aufweist.

2. Variante einer Eltern-Glucoamylase mit Glucoamylaseaktivität und verbesserter Wärmestabilität gegenüber der Eltern-Glucoamylase, wobei die Variante die Aminosäuresequenz von SEQ ID NO:2 aufweist und die Mutation A393R einschließt, oder wobei die Variante die Mutation in einer entsprechenden Position in einer homologen Glucoamylase aufweist, die einen Identitätsgrad von mindestens 70 % mit den in SEQ ID NO:2 gezeigten Aminosäuresequenzen aufweist.

3. Variante einer Eltern-Glucoamylase mit Glucoamylaseaktivität und verbesserter Wärmestabilität gegenüber der Eltern-Glucoamylase, wobei die Variante die Aminosäuresequenz von SEQ ID NO:2 aufweist und die Mutation Y402F einschließt, oder wobei die Variante die Mutation in einer entsprechenden Position in einer homologen Glucoamylase aufweist, die einen Identitätsgrad von mindestens 95 % mit den in SEQ ID NO:2 gezeigten Aminosäuresequenzen aufweist.

4. Variante nach einem der Ansprüche 1-3, wobei die homologe Eltern-Glucoamylase die Aspergillus niger G1-Glucoamylase ist.

5. Variante nach einem der Ansprüche 1-4, wobei die Glucoamylase eine, insbesondere am C-Terminus, verkürzte Glucoamylase ist.

6. DNA-Konstrukt, umfassend eine DNA-Sequenz, die eine Glucoamylasevariante nach einem der Ansprüche 1-5 codiert.

7. Rekombinanter Expressionsvektor, der ein DNA-Konstrukt nach Anspruch 6 trägt.

8. Zelle, die mit einem DNA-Konstrukt nach Anspruch 6 oder mit einem Vektor nach Anspruch 7 transformiert ist.

9. Zelle nach Anspruch 8, die ein Mikroorganismus, wie ein Bakterium oder ein Pilz, ist.

10. Zelle nach Anspruch 9, die ein Protease-defizienter Aspergillus oryzae oder ein Protease-defizienter Aspergillus niger ist.

11. Verfahren zur Umwandlung von Stärke oder teilweise hydrolysierter Stärke zu einem Sirup, der Dextrose enthält, wobei das Verfahren den Schritt der Saccharifizierung von Stärkehydrolysat in Gegenwart einer Glucoamylasevariante nach einem der Ansprüche 1-5 einschließt.

12. Verfahren nach Anspruch 11, wobei die Dosis an Glucoamylase im Bereich von 0,05 bis 0,5 AGU pro g Trockenmasse vorliegt.

13. Verfahren nach einem der Ansprüche 11 oder 12, umfassend die Saccharifizierung eines Stärkehydrolysats von mindestens 30 Gew.-% Trockenmasse.

14. Verfahren nach einem der Ansprüche 11-13, wobei die Saccharifizierung in Gegenwart eines Abbau-Enzyms durchgeführt wird, ausgewählt aus der Gruppe von Pullulanase und Isoamylase, vorzugsweise einer Pullulanase, die aus Bacillus acidopullulyticus oder Bacillus deramificans stammt, oder aus einer Isoamylase, die aus Pseudomonas amyloderamosa stammt.

15. Verfahren nach einem der Ansprüche 11-14, wobei die Saccharifizierung bei einem pH von 3 bis 5,5 und bei einer Temperatur von 60-80 °C, vorzugsweise von 63-75 °C, für 24 bis 72 h, vorzugsweise für 36-48 h bei einem pH von 4-4,5 durchgeführt wird.

16. Verfahren zur Saccharifizierung einer verflüssigten Stärkelösung, wobei das Verfahren umfasst
(i) einen Saccharifizierungsschritt, während welchem Schritt eine oder mehrere enzymatische Saccharifizierungsstadien stattfinden, und den anschließenden Schritt von
(ii) einem oder mehreren Hochtemperatur-Membrantrennschritten,
wobei die enzymatische Saccharifizierung unter Verwendung einer Glucoamylasevariante nach einem der Ansprüche 1-5 durchgeführt wird.

17. Verwendung einer Glucoamylasevariante nach einem der Ansprüche 1-5 bei einem Stärkeumwandlungsverfahren.

18. Verwendung einer Glucoamylasevariante nach einem der Ansprüche 1-5 bei einem kontinuierlichen Stärkeumwandlungsverfahren.

19. Verwendung nach Anspruch 18, wobei das kontinuierliche Stärkeumwandlungsverfahren ein kontinuierliches Saccharifizierungsverfahren nach Anspruch 16 umfasst.

20. Verwendung einer Glucoamylasevariante nach einem der Ansprüche 1-5 bei einem Verfahren zur Herstellung von Oligosacchariden.

21. Verwendung einer Glucoamylasevariante nach einem der Ansprüche 1-5 bei einem Verfahren zur Herstellung von Spezialsirupen.

22. Verwendung einer Glucoamylasevariante nach einem der Ansprüche 1-5 bei einem Verfahren zur Herstellung von Ethanol für Kraftstoff.

23. Verwendung einer Glucoamylasevariante nach einem der Ansprüche 1-5 bei einem Verfahren zur Herstellung eines Getränks.

24. Verwendung einer Glucoamylasevariante nach einem der Ansprüche 1-5 bei einem Fermentationsverfahren zur Herstellung von organischen Verbindungen, wie Citronensäure, Ascorbinsäure, Lysin, Glutaminsäure.

25. Verfahren zur Verbesserung der Wärmestabilität einer Eltern-Glucoamylase mit der in SEQ ID NO:2 gezeigten Aminosäuresequenz durch Vornehmen einer Mutation in einer oder mehreren der folgenden Position(en) der in SEQ ID NO:2 gezeigten Aminosäuresequenz:
E408R+S386N, T2Q+A11P+S394R, A11E+E408R, T2M+N9A+T390R+D406N+L410R, A393R, T2R+S386R+A393R, A393R+L410R, A1V+L66R+Y402F+N427S+S486G, T2E+T379A+S386K+A393R, Y402F, V59A+A393R+T490A und eine N-terminale Extension, bestehend aus PLASD, Y402F+S411 V, S 119P+Y402F+S411 V, S 119P+Y312Q+Y402F+T416H,
zur Verbesserung der Wärmestabilität einer Eltern-Glucoamylase, die einen Identitätsgrad von mindestens 60 % mit der in SEQ ID NO:2 gezeigten Aminosäuresequenz aufweist durch Vornehmen von einer oder mehreren Mutationen in einer entsprechenden Position in der homologen Glucoamylase.

## Revendications

1. Variant d'une glucoamylase parente ayant une activité glucoamylase et une thermostabilité améliorée par rapport à la glucoamylase parente, le variant ayant la séquence en acides aminés de SEQ ID NO:2 et comprenant une ou plusieurs mutation(s) à ou aux position(s) suivante(s) dans la séquence d'acides aminés montrée dans la SEQ ID NO: 2 E408R+S386N, T2Q+A11P+S394R, A11E+E408R, T2M+N9A+T390R+D406N+L410R, T2R+S386R+A393R, A393R+L410R, A1V+L66R+Y402F+N427S+S486G, T2E+T379A+S386K+A393R, V59A+A393R+T490A et une extension N-terminale consistant en PLASD, Y402F+S411V, S119P+Y402F+S411V, S119P+Y312Q+Y402F+T416H,
ou le variant ayant la ou les mutation(s) à une position correspondante dans une glucoamylase homologue qui montre un degré d'identité d'au moins 60 % avec les séquences d'acides aminés montrées dans SEQ ID NO: 2.

2. Variant d'une glucoamylase parente ayant une activité glucoamylase et une thermostabilité améliorée par rapport à la glucoamylase parente, le variant ayant la séquence en acides aminés de SEQ ID NO:2 et comprenant la mutation A393R,
ou le variant ayant la mutation à une position correspondante dans une glucoamylase homologue qui montre un degré d'identité d'au moins 70 % avec les séquences d'acides aminés montrées dans SEQ ID NO: 2.

3. Variant d'une glucoamylase parente ayant une activité glucoamylase et une thermostabilité améliorée par rapport à la glucoamylase parente, le variant ayant la séquence en acides aminés de SEQ ID NO:2 et comprenant la mutation Y402F,
ou le variant ayant la mutation à une position correspondante dans une glucoamylase homologue qui montre un degré d'identité d'au moins 95 % avec les séquences d'acides aminés montrées dans SEQ ID NO: 2

4. Variant selon l'une quelconque des revendications 1 à 3, dans lequel la glucoamylase parente homologue est la glucoamylase G1 d'*Aspergillus niger.*

5. Variant selon l'une quelconque des revendications 1 à 4, dans lequel la glucoamylase est une glucoamylase tronquée, en particulier en C-terminal.

6. Construction d'ADN comprenant une séquence d'ADN codant un variant glucoamylase selon l'une quelconque des revendications 1 à 5.

7. Vecteur d'expression recombinant qui porte une construction d'ADN selon la revendication 6.

8. Cellule qui est transformée avec une construction d'ADN selon la revendication 6 ou un vecteur selon la revendication 7.

9. Cellule selon la revendication 8, qui est un microorganisme, tel qu'une bactérie ou un champignon.

10. Cellule selon la revendication 9, qui est un *Aspergillus oryzae* ou un *Aspergillus niger* déficient en protéase

11. Procédé de conversion d'amidon ou d'amidon partiellement hydrolysé en un sirop contenant du dextrose, ledit procédé incluant l'étape de saccharification de l'hydrolysat d'amidon en présence d'un variant d'une glucoamylase selon l'une quelconque des revendications 1 à 5.

12. Procédé selon la revendication 11, dans lequel le dosage de glucoamylase est présent dans la gamme de 0,05 à 0,5 AGU par gramme de matière sèche.

13. Procédé selon l'une quelconque des revendications 11 ou 12, comprenant une saccharification d'un hydrolysat d'amidon d'au moins 30 % en poids de matière sèche.

14. Procédé selon l'une quelconque des revendications 1 1 à 13, dans lequel la saccharification est conduite en présente d'une enzyme débranchante choisie dans le groupe de la pullulanase et l'isoamylase, de préférence une pullulanase dérivée de *Bacillus acidopullulyticus* ou *Bacillus deramificans* ou une isoamylase dérivée de *Pseudomonas amyloderamosa.*

15. Procédé selon l'une quelconque des revendications 11 à 14, dans lequel la saccharification est conduite à un pH de 3 à 5,5 et à une température de 60 à 80°C, de préférence 63 à 75°C, pendant 24 à 72 heures, de préférence pendant 36 à 48 heures à un pH entre 4 et 4,5.

16. Méthode de saccharification d'une solution d'amidon liquéfié, la méthode comprenant :
(i) une étape de saccharification durant laquelle un ou plusieurs stades de saccharification enzymatique ont lieu, et l'étape ultérieure de
(ii) une ou plusieurs étapes de séparation par membrane à haute température,
dans laquelle la saccharification enzymatique est faite en utilisant un variant d'une glucoamylase selon l'une quelconque des revendications 1 à 5.

17. Utilisation d'un variant d'une glucoamylase selon l'une quelconque des revendications 1 à 5 dans un procédé de conversion d'amidon.

18. Utilisation d'un variant d'une glucoamylase selon l'une quelconque des revendications 1 à 5 dans un procédé de conversion d'amidon continu.

19. Utilisation selon la revendication 18, dans laquelle le procédé de conversion d'amidon continu inclut un procédé de saccharification continu selon la revendication 16.

20. Utilisation d'un variant d'une glucoamylase selon l'une quelconque des revendications 1 à 5 dans un procédé de production d'oligosaccharides.

21. Utilisation d'un variant d'une glucoamylase selon l'une quelconque des revendications 1 à 5 dans un procédé de production de sirops spécialisés.

22. Utilisation d'un variant d'une glucoamylase selon l'une quelconque des revendications 1 à 5 dans un procédé de production d'éthanol pour carburant.

23. Utilisation d'un variant d'une glucoamylase selon l'une quelconque des revendications 1 à 5 dans un procédé de production d'une boisson.

24. Utilisation d'un variant d'une glucoamylase selon l'une quelconque des revendications 1 à 5 dans un procédé de fermentation pour produire des composés organiques, tels que l'acide citrique, l'acide ascorbique, la lysine, l'acide glutamique.

25. Méthode pour améliorer la thermostabilité d'une glucoamylase parente ayant la séquence en acides aminés de SEQ ID NO:2 en faisant une mutation à une ou plusieurs des positions suivantes dans la séquence d'acides aminés montrée dans la SEQ ID NO: 2 : E408R+S386N, T2Q+A11P+S394R, A11E+E408R, T2M+N9A+T390R+D406N+L410R, A393R, T2R+S386R+A393R, A393R+L410R, A1V+L66R+Y402F+N427S+S486G, T2E+T379A+S386K+A393R, Y402F, V59A+A393R+T490A et une extension N-terminale consistant en PLASD, Y402F+S411V, S119P+Y402F+S411V, S 119P+Y312Q+Y402F+T416H
ou pour améliorer la thermostabilité d'une glucoamylase parente qui montre un degré d'identité d'au moins 60 % avec les séquences d'acides aminés montrées dans SEQ ID NO: 2 en faisant la ou les mutation(s) à une position correspondante dans une glucoamylase homologue.
